(19) Europäisches Patentamt

European Patent Office

Office européen des brevets

(11) **EP 0 182 152 B1**

(12) **EUROPEAN PATENT SPECIFICATION**

(45) Date of publication and mention
of the grant of the patent:
**26.05.1999 Bulletin 1999/21**

(51) Int Cl.$^6$: **C12P 1/06**, C07G 11/00,
A61K 35/66, C12N 1/20
// (C12P1/06, C12R1:29,
C12N1:20, C12R1:29)

(21) Application number: 85113751.3

(22) Date of filing: **29.10.1985**

(54) **Antitumor antibiotics (LL-E33288 Complex)**

Antitumorale Antibiotika (Komplex LL-E-33288)

Antibiotiques antitumoraux (complexe LL-E33288)

(84) Designated Contracting States:
**AT BE CH DE FR GB IT LI NL SE**

(30) Priority: **16.11.1984 US 672031**
**17.10.1985 US 787066**

(43) Date of publication of application:
**28.05.1986 Bulletin 1986/22**

(73) Proprietor: **AMERICAN CYANAMID COMPANY**
**Wayne, NJ 07470-8426 (US)**

(72) Inventors:
• **Lee, May Dean-Ming**
**Monsey New York 10952 (US)**
• **Greenstein, Michael**
**Suffern New York 10901 (US)**
• **Labeda, David Paul**
**Peoria Illinois 61614 (US)**

(74) Representative:
**Wächtershäuser, Günter, Prof. Dr.**
**Patentanwalt,**
**Tal 29**
**80331 München (DE)**

(56) References cited:
• **CHEMICAL ABSTRACTS, vol. 103, no. 9, 2nd**
**September 1985, page 525, abstract no. 69796b,**
**Columbus, Ohio, US; & JP-A-60 24 195 (KYOWA**
**HAKKO KOGYO CO., LTD) 06-02-1985**
• **CHEMICAL ABSTRACTS, vol. 102, no. 23, 10th**
**June 1985, page 341, abstract no. 200941u,**
**Columbus, Ohio, US; M.S. PUAR et al.: "The**
**biosynthesis of hazimycins: possible origin of**
**isonitrile carbon", & J. ANTIBIOT. 1985, 38(4),**
**530-2**
• **CHEMICAL ABSTRACTS, vol. 101, no. 13, 24th**
**September 1984, page 497, abstract no. 108881f,**
**Columbus, Ohio, US; M. ZHAO et al.: "Studies on**
**sagamicin produced by M. echinospora mutant**
**strain. II. Selection and fermentation of M.**
**echinospora JIM-401", & KANGSHENGSU 1984,**
**9(2), 90-3**
• **CHEMICAL ABSTRACTS, vol. 96, 1982, pages**
**278-279, abstract no. 118046e, Columbus, Ohio,**
**US; Y. IKEDA et al.: "Dotriacolide, a new**
**beta-lactamase inhibitor", & J. ANTIBIOT. 1981,**
**34(12), 1628-30**
• **CHEMICAL ABSTRACTS, vol. 94, 1981, page 396,**
**abstract no. 99356w, Columbus, Ohio, US; H.**
**MAEHR et al.: "Microbial products. IV, X-14847,**
**a new amynoglycoside from Micromonospora**
**echinospora", & J. ANTIBIOT. 1980, 33(12),**
**1431-6**
• **CHEMICAL ABSTRACTS, vol. 92, 1980, page 502,**
**abstract no. 74356s, Columbus, Ohio, US; &**
**HU-A-17 055 (CHINOIN GYOGYSZER ES**
**VEGYESZETI TERMEKEK GYARA Rt.) 28-09-79**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to new antibacterial and antitumor agents designated LL-E3328$\alpha_1$-Br, LL-E33288 $\alpha_1$-I LL-E33288$\alpha_2$-Br, LL-E33288$\alpha_2$-I, LL-E33288$\alpha_3$-Br, LL-E33288$\alpha_3$-I, LL-E33288$\alpha_4$-Br, LL-E33288$\beta_1$-Br, LL-E33288$\beta_1$-I, LL-E33288 $\beta_2$-Br, LL-E33288$\beta_2$-I, LL-E33288$\gamma_1$-Br, LL-E33288$\gamma_1$-I and LL-E33288$\delta_1$-I, to their production by fermentation, to methods for their recovery and concentration from crude solutions and to processes for their purification. The present invention includes within its scope the antibacterial and antitumor agents in dilute form, as crude concentrates, as a complex of various or all components, in pure form as individual components and novel strains of <u>Micromonospora</u>.

The LL-E33288 antibiotics of this invention are closely related compounds. The fourteen antibiotics are recovered from fermentation and are initially obtained as a mixture, hereinafter either the LL-E3888 complex, the the LL-E33288 Iodo-complex or the LL-E33288 Bromo-complex. In general, the iodine containing components of the LL-E33288 antibiotics <u>(e.g.</u>, $\alpha_1$-I, $\alpha_2$-I, $\alpha_3$-I, $\beta_1$-I, $\beta_2$-I, $\gamma_1$-I and $\delta_1$-I) are found only in fermentations using media containing inorganic or organic iodide while the bromine containing components <u>(e.g.</u>, $\alpha_1$-Br, $\alpha_2$-Br, $\alpha_3$-Br, $\alpha_4$-Br, $\beta_1$-Br, $\beta_2$-Br and $\gamma_1$-Br) are found only in fermentations using media containing inorganic or organic bromide. The ratio of components in the LL-E3388 complex will vary, depending upon the fermentation of both the bromine and the iodine containing antibiotics, LL-E33288$\beta_1$ and LL-E33288$\gamma_1$ are the major components, together accounting for approximately 90% of the complex. LL-E33288$\alpha_1$, LL-E33288$\alpha_2$, LL-E33288$\alpha_3$, LL-E33288$\alpha_4$-Br, LL-E33288$\beta_2$ and LL-E33288$\delta_1$-I are minor components, together accounting for approximately 10% of the complex.

The LL-E33288 antibiotics are active against gram-positive and gram-negative bacteria. Each of the components were also found to be active in a modification of the Biochemical Induction Assay [Elespuru, R. and Yarmolinsky, M., Environmental Mutagenesis, 1, 65-78 1979)]. a test which specifically measures the ability of an agent to directly or indirectly initiate DNA damage. In this assay, both LL-E33288$\beta_1$-Br and LL-E33288$\gamma_1$-Br were active at concentrations lower than $1 \times 10^{-6}$ mcg/ml.

## BRIEF DESCRIPTION OF THE DRAWINGS

FIG. I is the ultraviolet absorption spectra of LL-E33288$\beta_1$-Br;
FIG. II is the infrared absorption spectrum of LL-E33288$\beta_1$-Br;
FIG. III is the proton magnetic resonance spectrum of LL-E33288$\beta_1$-Br;
FIG. IV is the carbon 13 magnetic resonance spectrum of LL-E33288 $\beta_1$-Br;
FIG. V is the ultraviolet absorption spectra of LL-E33288$\gamma_1$-Br;
FIG. VI is the infrared absorption spectrum of LL-E33288$\gamma_1$-Br;
FIG. VII is the proton magnetic resonance spectrum of LL-E33288 $\gamma_1$-Br;
FIG. VIII is the carbon 13 magnetic resonance spectrum of LL-E33288$\gamma_1$-Br;
FIG. IX is the proton magnetic resonance spectrum of LL-E33288 $\alpha_2$-I;
FIG. X is the proton magnetic resonance spectrum of LL-E33288 $\alpha_3$-I;
FIG. XI is the ultraviolet absorption spectra of LL-E33288$\beta_1$-I;
FIG. XII is the infrared absorption spectrum of LL-E33288$\beta_1$-I;
FIG. XIII is the protein magnetic resonance spectrum of LL-E33288$\beta_1$-I;
FIG. XIV is the carbon 13 magnetic resonance spectrum of LL-E33288$\beta_1$-I;
FIG. XV is the ultraviolet absorption spectra of LL-E33288$\gamma_1$-I;
FIG. XVI is the infrared absorption spectrum of LL-E33288$\gamma_1$-I;
FIG. XVII is the proton magnetic resonance spectrum of LL-E33288$\gamma_1$-I; and
FIG. XVIII is the carbon 13 magnetic resonance spectrum of LL-E33288$\gamma_1$-I.

## DETAILED DESCRIPTION OF THE INVENTION

The physico-chemical characteristics of LL- E33288 $\beta_1$-Br and LL-E33288$\gamma_1$-Br are described below:
<u>LL-E33288$\beta_1$-Br</u>

1) Approximate elemental analysis: C 48.6; H 5.6; N 2.9; S 9.1; and Br 5.5. (It has been determined by electron spectroscopy for chemical analysis (ESCA) that only the following elements are present: C, H, N, O, S and Br);
2) Melting point: 146-150°C (dec.);
3) Specific rotation: $[\alpha]_D^{26}$ = -49±10°(0.1% ethanol);
4) Ultraviolet absorption spectra: as shown in Figure I (methanol; acidic methanol; basic methanol);
5) Infrared absorption spectrum: as shown in Figure II (KBr disc);

6) Proton magnetic resonance spectrum: as shown in Figure III (300 MHz, $CDCl_3$);

7) Carbon-13 magnetic resonance spectrum: as shown in Figure IV (75.43 MHz, $CDCl_3$, ppm from TMS), significant peaks as listed below:

| | | | |
|---|---|---|---|
| 17.60(q); | 17.64(q); | 18.9(q); | 19.7(q); |
| 22.4(q); | 22.8(q); | 23.5(q); | 34.3(t): |
| 36.9(t); | 39.2 (t/d); | 47.8(d); | 51.7(q); |
| 52.7(q); | 54.6 (t/d); | 56.3(q); | 57.2(q); |
| 57.8(d); | 61.0(q/d); | 61.7(d); | 62.4(t); |
| 66.9(d); | 68.4(d); | 69.1(d); | 69.7(d); |
| 70.2(d); | 71.1(d); | 71.9(d); | 72.1(s/t); |
| 76.1(d); | 81.0(d); | 83.3(s); | 88.2(s); |
| 97.4(d); | 99.7(d); | 100.8(s); | 102.5(d); |
| 115.1(s); | 123.4(d); | 124.4(d); | 126.5(d); |
| 130.2(s); | 130.8(s); | 144.6(s); | 149.3(s); |
| 149.5(s); | 191.7(s); | 192.4(s); | |

8) Molecular weight: 1333/1335 respectively for $^{79}$Br/[81] Br as determined by FAB-MS; and

9) Molecular formula: $C_{54}H_{84}N_3O_{22}S_4Br$, exact masses at 1258.3699 ($^{79}$Br) and 1260.3726($^{81}$Br) was determined by high resolution FAB-MS and calculated to be $C_{52}H_{81}N_3O_{21}S_3Br$ (M+H-$C_2H_4OS$).

<u>LL-E33288 $\gamma_1$-Br</u>

1) Ultraviolet absorption spectra: as shown in Figure V (methanol; acidic methanol; basic methanol);

2) Infrared absorption spectrum: as shown in Figure VI (KBr disc):

3) Proton magnetic resonance spectrum: as shown in Figure VII (300 MHz, $CDCl_3$);

4) Carbon 13 magnetic resonance spectrum: as shown in Figure VIII (75.43 MHZ, $CDCl_3$, ppm from TMS), significant peaks as listed below:

| | | | |
|---|---|---|---|
| 14.4 | 17.6 | 17.9 | 19.0 |
| 19.7 | - | 22.8 | - |
| - | 34.0 | 37.6 | 39.5 |
| 42.1 | - | 51.6 | 52.7 |
| 54.1 | 56.3 | 57.3 | - |
| 59.3 | 61.1 | 61.8 | 61.9 |
| 67.2 | 68.18 | 68.23 | 69.7 |
| 70.1 | 70.8 | 71.1 | 71.7 |
| 71.8 | 76.1 | - | 81.0 |
| 82.9 | 88.4 | - | 97.8 |
| 100.0 | 100.2 | 101.3 | 103.0 |
| 115.3 | 123.0 | 124.9 | 126.9 |
| 130.4 | 131.1 | 131.8 | 138.0 |
| 144.7 | - | 149.5 | 149.6 |
| 155.6 | 192.5 | 192.9 | |

5) Molecular formula: $C_{53}H_{82}N_3O_{22}S_4Br$ by comparing its UV, IR, 'H NBR, and $^{13}$C NMR data to those of LL-E33288 $\beta_1$-BR and LL-E33288 $\gamma_1$-I; and

6) Molecular weight: 1319/1321 respectively for $^{79}$Br/[81] Br, calculated from its molecular formula.

The physico-chemical characteristics of LL-E33288$\alpha_1$-I, LL-E33288$\alpha_2$-I, LL-E33288$\alpha_3$-I, and LL-E33288 $\beta_1$-I and LL-E33288$\gamma_1$ -I are described below:

<u>LL-E33288$\alpha_1$-I</u>

1) Molecular weight: 1145, determined by FAB-MS.

LL-E33288$\alpha_2$-I

1) Contains and only contains the following elements by electron spectroscopy for chemical analysis (ESCA): C, H, N, O, S, I;
2) Molecular weight: 1131, determined by FAB-MS; and
3) Proton magnetic resonance spectrum: as shown in Figure IX (300 MHz, $CDCl_3$).

LL-E33288 $\alpha_3$-I

1) Molecular weight: 1066, determined by FAB-MS; and
2) Proton magnetic resonance spectrum: as shown in Figure X (300 MHz, $CDCl_3$).

LL-E33288 $\beta_1$-I

1) Ultraviolet absorption spectra: as shown in Figure XI (methanol; acidic methanol; basic methanol);
2) Infrared absorption spectrum: as shown in Figure XII (KBr disc);
3) Proton magnetic resonance spectrum: as shown in Figure XIII (300 MHz, $CDCl_3$);
4) Carbon 13 magnetic resonance spectrum: as shown in Figure XIV (75.43 MHz, $CDCl_3$, ppm from TMS), significant peaks as listed below:

| - | 17.5 | 17.6 | 18.9 |
|---|---|---|---|
| - | 22.4 | 22.8 | 23.4 |
| 25.4 | 34.3 | 36.9 | 39.2 |
| - | 47.9 | 51.6 | 52.8 |
| 54.8 | 56.3 | 57.2 | 57.9 |
| 60.9 | | 61.6 | 62.2 |
| 67.0 | 68.4 | 68.4 | 69.1 |
| 69.6 | 70.4 | 71.1 | 71.8 |
| 72.2 | 76.2 | - | 80.8 |
| 83.3 | 88.1 | 93.6 | 97.4 |
| 99.6 | 99.6 | - | 102.6 |
| 112.4 | 123.4 | 124.4 | 126.4 |
| - | - | 133.4 | - |
| - | - | - | - |
| - | 192.3 | 192.6 | |

5) Molecular formula: $C_{54}H_{84}N_3O_{22}S_4I$ by comparing its UV, IR, $^1$H NMR and $^{13}$C NMR data to those of LL-E33288 $\beta_1$-Br and LL-E33288 $\gamma_1$-I; and
6) Molecular weight: 1381, calculated from molecular formula.

LL-E33288 $\gamma_1$-I

1) Contains and only contains the following elements by electron spectroscopy for chemical analysis (ESCA): C, H,N,O,S,I;
2) Approximate elemental analysis: C 48.8; H 5.4; N 2.8; S 9.0; I 9.2;
3) Molecular weight: 1367, determined by FAB-MS;
4) Molecular formula: $C_{53}H_{82}N_3O_{22}S_4I$, exact mass for M+H was determined by high resolution FAB-MS to be 1368. 3397 for $C_{53}H_{83}N_3O_{22}S_4I$;
5) Ultraviolet absorption spectra: as shown in Figure XV (methanol; acidic methanol; basic methanol);
6) Infrared absorption spectrum: as shown in Figure XVI (KBr disc);
7) Proton magnetic resonance spectrum: as shown in Figure XVII (300 MHz, $CDCl_3$); and
8) Carbon 13 magnetic resonance spectrum: as shown in Figure XVIII (75.43 MHz, $CDCl_3$, ppm for TMS) significant peaks as listed below:

| 14.5(q) | 17.6(q) | 17.6(q) | 18.9(q) |
|---|---|---|---|

(continued)

| - | - | 22.8(q) | - |
|---|---|---|---|
| 25.4(q) | 34.1(t) | 37.0(t) | 39.1(t) |
| 42.3(t/s) | - | 51.5(d) | 52.8(q) |
| 54.8(t) | 56.3(q) | 57.2(q) | - |
| 60.4(d) | 60.9(q) | 61.3(t) | 61.7(q) |
| 67.0(d) | 68.4(d) | 68.5(d) | 69.2(d) |
| 69.7(d) | 70.5(d) | 71.1(d) | 71.8(d) |
| 72.1(s) | 75.7(d) | 75.8(d) | 80.9(d) |
| 82.8(s) 99.6(d) | 88.1(s) 99.7(d) | 93.5(s) 100.8(s) | 97.3(d) 102.6(d) |
| - | 123.4(d) | 124.4(d) | 126.2(d) |
| 130.2(s) | 131.0(s) | 133.4(s) | 139.1(s) |
| 143.0(s) | 145.1 | 150.6(s) | 151.5(s) |
| 154.5 | 192.0(s) | 192.5(s) | |

The LL-E33288 components are most conveniently separated and identified by high-performance liquid chromatography (HPLC) and by thin-layer chromatography (TLC). It is difficult, although not impossible, to separate the corresponding iodinated and brominated components by HPLC; however, they cannot be distinquished by TLC.

The preferred analytical separation of the LL-E33288-Br components by HPLC uses the following conditions:

| Column: | "Separalyte $C_{18}$ 5 m," 4.6 mm x 25 cm (Analytichem International); |
|---|---|
| Solvent: | Acetonitrile: 0.2$\underline{M}$ aqueous ammonium acetate (60:40); |
| Flow rate: | 1.5 ml/minute |
| Detector: | Dual wavelength UV at 254 nm and 280 nm; |
| Sensitivity: | 0-0.02 A.U.F.S. |

Table IA gives the approximate retention times and volumes of LL-E33288$\beta_1$-Br, LL-E33288$\beta_2$-Br, and LL-E33288 $\gamma_1$-Br under these conditions.

TABLE IA

| LL-E33288 Components | Retention Time (minutes) | Retention Volume(ml) |
|---|---|---|
| $\beta_1$-Br | 5.7 | 8.6 |
| $\beta_2$-Br | 7.1 | 10.7 |
| $\gamma_1$-Br | 4.3 | 6.5 |

The preferred analytical HPLC separation of the iodine containing LL-E33288 components uses the following conditions:

| Column: | NOVA-PAK $C_{16}$ Radial-PAK cartridge with RCM-100 Radial Compression Module (Millipore, Waters Chromatography Division); |
|---|---|
| Solvent: | Acetonitrile: 0.2$\underline{M}$ aqueous ammonium acetate (50:50); |
| Flow Rate: | 1.2 ml/minute; |
| Detector: | Dual wavelength UV at 254 nm and 280 nm; |

(continued)

| Sensitivity: | 0-0.02 A.U.F.S. |
|---|---|

Table IB gives the approximate retention times and volumes of LL-E33288 $\alpha_1$-I, LL-E33288$\alpha_2$-I, LL-E33288$\alpha_3$-I, LL-E33288 $\beta_1$-I, LL-E33288 $\beta_2$-I, LL-E33288 $\gamma_1$-I and LL-E33288$\delta_1$-I under these conditions.

TABLE IB

| LL-E33288 Components | Retention Time (minutes) | Retention Volume(ml) |
|---|---|---|
| $\alpha_1$-I | 11.9 | 14.3 |
| $\alpha_2$-I | 9.1 | 10.9 |
| $\alpha_3$-I | 1.5 | 1.8 |
| $\beta_1$-I | 4.4 | 5.3 |
| $\beta_2$-I | 5.0 | 6.0 |
| $\gamma_1$-I | 3.6 | 4.3 |
| $\delta_1$-I | 2.6 | 3.1 |

The LL-E33288 components are separated and identified by the following TLC system:

| Adsorbant: | Silica gel 60 $F_{254}$ pre-coated aluminum sheets, 0.2mm layer thickness, EM Reagents; | |
|---|---|---|
| Detection: | Visualized by quenching effect under short wavelength UV lamp (254 nm), and bioautography using <u>Bacillus</u> <u>subtilis</u> or the modified biochemical induction assay; | |
| Solvent Systems: | I, | ethyl acetate saturated with 0.1<u>M</u> aqueous potassium dihydrogen phosphate; |
| | II, | 3% isopropyl alcohol in ethyl acetate saturated with 0.1<u>M</u> aqueous potassium dihydrogen phosphate; |
| | III, | ethyl acetate:methanol (95.5). |

Table II gives the approximate Rf values of LL-E33288 components in these three systems:

TABLE II

| LL-E33288 Components | $R_f$ Value | | |
|---|---|---|---|
| | Solvent System I | Solvent System II | Solvent System III |
| $\alpha_1$-Br,$\alpha_1$-I | 0.67 | 0.80 | 0.79 |
| $\alpha_2$-Br,$\alpha_2$-I | 0.61 | 0.75 | 0.73 |
| $\alpha_3$-Br,$\alpha_3$-I | 0.55 | 0.69 | 0.61 |
| $\alpha_4$-Br | 0.49 | 0.64 | 0.54 |
| $\beta_2$-Br,$\beta_2$-I | 0.32 | 0.41 | 0.45 |
| $\beta_1$-Br,$\beta_1$-I | 0.24 | 0.35 | 0.36 |
| $\gamma_1$-Br,$\gamma_1$-I | 0.18 | 0.28 | 0.27 |

TABLE II   (continued)

| LL-E33288 Components | R_f Value | | |
| --- | --- | --- | --- |
| | Solvent System I | Solvent System II | Solvent System III |
| $\delta_1$-I | 0.11 | 0.19 | |

The new antibacterial and antitumor agents designated LL-E33288$\alpha_1$-Br, LL-E33288$\alpha_2$-Br, LL-E33288$\alpha_3$-Br, LL-E33288$\alpha_4$-Br, LL-E33288 $\beta_1$-Br, LL-E33288$\beta_2$-Br, LL-E33288 $\gamma_1$-Br, LL-E33288$\alpha_1$-I, LL-E33288$\alpha_2$-I, LL-E33288 $\alpha_3$-I, LL-EE288$\beta_1$-I, LL-E33288$\beta_2$-I, LL-E33288$\gamma_1$-I and LL-E33288$\delta_1$-I are formed during the cultivation under controlled conditions of a new strain of Micromonospora echinospora ssp. calichensis. This microorganism is maintained in the culture collection of the Medical Research Division, American Cyanamid Company, Pearl River, New York as culture number LL-E33288. A viable culture of this new microorganism has been deposited with the Culture Collection Laboratory, Northern Regional Research Center, U. S. Department of Agriculture, Peoria, Illinois on August 9, 1984, and has been added to its permanent collection. It has been assigned by such depository the strain designation NRRL 15839. Access to such culture, under strain designation NRRL 15839, during pendency of the instant application shall be available to one determined by the Commissioner of Patents and Trademarks to be entitled thereto under 37 C.F. R. §1.14 and 35 U.S.C. §122, and all restrictions on availability to the public of such culture will be irrevocably removed upon grant of a patent on the instant application.

Culture LL-E33288 was isolated from a caliche clay soil sample collected in Texas.

The generic assignment of the strain NRRL 15839 to the genus Micromonospora was confirmed morphologically and chemically. The strain produces monospores either singly or in masses on the vegetative hyphae. No aerial hyphae were observed. Electron microscopic examination showed that the spores were warty. Whole cell analysis showed that the strain contained the meso isomer of diaminopimelic acid (DAP). The 3-OH derrivative of DAP was present in large (major) amounts. Additionally the strain showed the presence of xylose plus traces of arabinose in its whole cell sugar hydrolysates (whole cell sugar pattern of Type D).

From macromorphological and physiological studies it was concluded that NRRL 15839 can be considered subspecies of M. echinospora (it is closest to M. echinospora ssp. pallida). Data on the morphology of NRRL 15839 are given in Tables A and B. Physiological data are given in Tables C and D.

Table A

| Macromorphology Of NRRL 15839 (Colors Are NBS-ISCC) | | | |
| --- | --- | --- | --- |
| ISP Agar Medium | Spores | Vegetative Mycelium | Soluble Pigments |
| Yeast-Malt (ISP 2) | - | Dark orange-yellow (72) | |
| Oatmeal (ISP 3) | - | Colorless → pale orange-yellow (73) | - |
| Inorganic Salts-Starch (ISP 4) | Slight border of black spores | Dark orange-yellow (72) to lt. yellow-brown (76) | Lt. brownish |
| Glycerol-Asparagine (ISP 5) | - | Pale orange-yellow (73) → colorless | - |

Table B

| Macromorphology of NRRL-15839 on Various Agar Media Used for Actinomycete Growth (28°C, 2 weeks) | |
| --- | --- |
| Agar Medium | NRRL-15839 |
| Pablum | Beige veg. Sl. black spores No sol. pig. |
| Yeast Czapeks | Beige veg. No spores No sol. pig. |
| Czapek's | Beige veg. Sl. black spores No sol. pig. |

Table B   (continued)

| Macromorphology of NRRL-15839 on Various Agar Media Used for Actinomycete Growth (28°C, 2 weeks) | |
| --- | --- |
| Agar Medium | NRRL-15839 |
| Yeast Dextrose | Tan veg. Moderate black sp. Sl. dark pig. |
| Nutrient | Colorless to can veg. Sl. black spores No sol. pig. |
| Nutrient-Glycerol | Colorless to light beige veg. No black spores No sol. pig. |
| Bennett's Dextrin | Colorless to beige veg. Sl. black spores Sl. rosy-brown pig. |
| Glucose-Asparagine | Colorless to lt. orange-beige veg. No spores No sol. pig. |
| veg. = vegetative hyphae; pig. = pigment. | |

Table C

| Carbohydrate Utilization of NRRL-15839 | |
| --- | --- |
| Arabinose | + |
| Cellulose | - |
| Fructose | + |
| Glucose | + |
| Inositol | - |
| Mannitol | - |
| Raffinose | ± |
| Rhamnose | + |
| Sucrose | + |
| Xylose | + |

Table D

| Physiological Reactions of NRRL-15839 | |
| --- | --- |
| Hydrolysis of | |
| Casein | + |
| Xanthine | - |
| Hypoxanthine | - |
| Tyrosine | + |
| Adenine | - |
| Gelatin | + |
| Potato Starch | + |

Table D   (continued)

| Physiological Reactions of NRRL-15839 | |
|---|---|
| Hydrolysis of | |
| Esculin | + |
| Production of | |
| Nitrate Reductase | + |
| Phospatase | W |
| Urease | - |
| Growth on | |
| Salicin | - |
| 5% NaCl | - |
| Lysozyme Broth | - |
| Decarboxylation of | |
| Acetate | + |
| Benzoate | - |
| Citrate | - |
| Lactate | - |
| Malate | - |
| Mucate | - |
| Oxalate | - |
| Propionate | + |
| Pyruvate | + |
| Succinate | - |
| Tartrate | - |
| Acid from | |
| Adonitol | - |
| Arabinose | + |
| Cellobiose | + |
| Dextrin | + |
| Dulcitol | - |
| Erythritol | - |
| Fructose | + |
| Galactose | V |
| Glucose | + |
| Glycerol | - |
| Inositol | - |
| Lactose | - |
| Maltose | + |
| Mannitol | - |
| Mannose | + |
| $\alpha$-methyl D Glucoside | - |
| Melibiose | - |
| Raffinose | + |
| Rhamnose | + |

Table D   (continued)

| Physiological Reactions of NRRL-15839 | |
|---|---|
| Acid from | |
| Salicin | + |
| Sorbitol | - |
| Sucrose | + |
| Trehalose | + |
| Xylose | + |
| β-Methyl D-xyloside | - |
| Growth at | |
| 10° | - |
| 42° | + |
| 45° | + |
| + = positive; - = negative; V = variable; W = weak. | |

Derivation of Mutant LL-E33288-R66, NRRL-15975

In an effort to improve fermentation yields, the orginal culture LL-E33288 (NRRL-15839) was plated and 50 single colonies were isolated. These were designated NS1 to NS50 (NS = natural selection).

Fermentation of these isolates showed that those with moderate sporulation were generally better producers of LL-E33288 complex. Selected as representative of this group was isolate NS6.

Using isolate NS6 as the starting culture, spore suspensions were prepared and exposed to various mutagens. Single colonies were isolated from a nitroso guanidine treatment, but none proved to be significantly improved producers of the LL-E33288 complex. From a subsequent series of exposures to ultraviolet irradiation, single colonies were obtained from which isolate UV 610 was selected as a high yielding mutant. Isolate UV 610 was then streaked and sub-isolates 1 to 7 were obtained. Sub-isolate UV 610(3) was selected for further work.

Because of the highly potent antibacterial and antineoplastic nature of the LL-E33288 complex it is possible that once a limited concentration of the antibiotic is biosynthesized in the fermentation it may become toxic/inhibitory to the producing culture. Thus, an effort was made to obtain isolates which are resistant to the LL-E33288 antibiotic complex.

Vegetative growth from isolate UV 610(3) was prepared as employed for fermentation and used to inoculate a flask of medium consisting of peptone, dextrose, molasses and water. The medium was supplemented with LL-E33288$\beta_1$-Br at a concentration of 8 $\mu$g/m. A number of platings were done from this flask and a resistant population was obtained on the seventh day. A total of 97 colonies (R1 to R97) were isolated. Isolate R66 was selected as a potentially improved producer of LL-E33288 $\beta_1$-Br.

The history is represented schematically below.

```
                         E33288 (Wild type)




                         NS6 (Natural selection)



                         |----NTG [NG1-NG120](Nitrosoguanidine)



                         |----UV [UV1-UV200](Ultraviolet)



                         |----UV [UV201-UV400](Ultraviolet)



                         |----UV [UV401-UV702](Ultraviolet)



                              UV610(3)(Sub-islate 3)



                         [R1 to R97](Resistant colonies)



                              R66
```

The mutant R66 is maintained in the culture collection of the Medical Research Division, American Cyanamid Company, Pearl river, New York as culture number LL-E33288 R66. A viable culture of this new microorganism has been deposited with the Culture Collection Laboratory, Northern Regional Research center, U. S. Department of Agriculture, Peoria, Illinois on June 6, 1985, and has been added to its permanent collection. It has been assigned by such depository the strain designation NRRL 15975. Access to such culture, under strain designation NRRL 15975, during pendency of the instant application shall be available to one determined by the Commissioner of Patents and Trademarks to be entitled thereto under C.F.R. §1.14 and 35 U.S.C. §122, and all restrictions on availability to the public of such culture will be irrevocably removed upon grant of a patent on the instant application.

Morphologically, NRRL-15975 forms fewer spores than NRRL-15839. A comparison of NRRL-15975 with NRRL-15839 is given in Table DD.

Chemically, both NRRL-15839 and NRRL-15975 show the same whole cell sugar patterns (Type D: xylose and traces of arabinose). The whole cell diaminopimelic acid analysis reveals that 15975 does not form the meso isomer but only the 3-hydroxy derivative (NRRL-15839 contains both compounds). This does not change the chemotaxonomic assignment.

Physiological tests show that NRRL-15839 and NRRL-15975 differ in only two physiological reaction (See Table D). NRRL-15975 is negative for nitrate reductase and positive for utilization of lactate. NRRL-15839 was weakly positive for both, but is now negative after having been maintained on slants for a few months. Thus these characters should be considered variable for this taxon.

EP 0 182 152 B1

TABLE DD

| Morphological Comparison of NRRL 15839 and NRRL 15975 | | | |
|---|---|---|---|
| Agar Medium | | NRRL 15839 | NRRL 15975 |
| Bennett's-Dextrin | V[1] | Beige-can | Beige can |
| | Sp | Black, copious | None |
| | SS | None | None |
| Czapek's | V Sp | Orange-tan Blac , traces | Orange-can None |
| | SS | None | None |
| Yeast Extract-Czapek's | V Sp | Orange tan, flat Black, traces | Orange tan, convoluted None |
| | SS | None | Slight yellowish |
| Potato-Dextrose | V | Very poor growth | Very poor growth |
| | Sp | None | None |
| | SS | None | None |
| Nutrient glycerol | V | Tan | Tan |
| | Sp SS | Black, sparse None | Black, sparse Slight brownish |
| Nutrient | V | Tan | Tan |
| | Sp | Black, fair | None |
| | SS | None | None |
| 1 = V = vegetative hyphae; Sp = spores; SS = soluble pigment. | | | |

It is to be understood that for the production of these new antibacterial and antitumor agents the present invention is not limited to this partucular organisms or to organisms fully answering the above growth microscropic characteristics which were given for illustrative purposes only. In fact, it is desired and intended to include the use of mutants produced from these organisms by various means such as exposure to X-radiation, ultraviolet radiation, N'-methyl-N'-nitro-N-nitrosoguanidine, actinophages and the like.

The in vitro antibacterial activity of LL-E33288 components was determined against a spectrum of gram-positive and gram-negative bacteria by a standard agar dilution method. Mueller-Hinton again containing two-fold decreasing concentrations of the antibiotics were poured into petri plates. the agar surfaces were inoculated with 1 to 5 x $10^4$ colong forming units of bacteria by means of the steers replicating device. The lowest concentration of LL-E33288 component that inhibited growth of a bacterial strain after about 18 hours of incubation at approximately 35°C was recorded as the minimal inhibitory concentration (MIC) for that strain. The results are summarized in Table III.

## TABLE III

### In vitro Antibacterial Activity of LL-E33288 Components

| Organism | | Minimal Inhibitory Concentration, mcg/ml | | | |
|---|---|---|---|---|---|
| | | $\beta_1$-Br | $\beta_1$-I | $\gamma_1$-Br | $\gamma_1$-I |
| Escherichia coli | CMC 84-11 | 0.25 | 0.50 | 0.50 | 0.25 |
| " " | No. 311-(MP) | 0.12 | 0.25 | 0.25 | 0.25 |
| " " | ATCC 25922 | 0.12 | 0.25 | 0.25 | 0.25 |
| Klebsiella pneumoniae | CMC 84-5 | 0.25 | 0.50 | 0.50 | 0.25 |
| " " | AD (MP) | 0.12 | 0.50 | 0.50 | 0.25 |
| Enterobacter cloacae | CMC 84-4 | 0.5 | 0.50 | 0.50 | 0.50 |
| " aerogenes | IO 83-44 | 0.25 | 0.25 | 0.50 | 0.50 |
| Serratia marcescens | CMC 83-27 | 0.12 | 0.25 | 0.50 | 0.25 |
| " " | F35 (MP) | 0.12 | 0.50 | 0.25 | 0.12 |
| Morganella morganii | 10 83-18 | 0.5 | 1 | 0.50 | 0.25 |
| Providencia stuartii | CMC 83-82 | 0.25 | 0.50 | 1 | 0.25 |
| Citrobacter diversus | K 82-84 | 0.12 | 0.50 | 0.50 | 0.25 |
| " freundii | IO 83-13 | 0.12 | 0.25 | 0.25 | 0.12 |
| Acinetobacter sp. | CMC 83-89 | 0.06 | 0.25 | 0.25 | 0.12 |
| " sp. | IO 83-49 | 0.12 | 0.25 | 0.25 | 0.06 |

EP 0 182 152 B1

TABLE III (continued

| Organism | | Minimal Inhibitory Concentration, mcg/ml | | | |
|---|---|---|---|---|---|
| | | $\beta_1$-Br | $\beta_1$-I | $\gamma_1$-Br | $\gamma_1$-I |
| Pseudomonas aeruginosa | 12-4-4 (MP) | 0.5 | 0.50 | 1 | 0.25 |
| " " | ATCC 27853 | 0.25 | 0.25 | 0.50 | 0.12 |
| Staphylococcus aureus | Smith | ≤ 0.0025 | ≤ 0.000031 | ≤ 0.000031 | ≤ 0.000031 |
| " " | SSC 82-31 | ≤ 0.00025 | ≤ 0.000031 | ≤ 0.000031 | ≤ 0.000031 |
| " " | ATCC 25923 | ≤ 0.00025 | ≤ 0.000031 | ≤ 0.000031 | ≤ 0.000031 |
| " " | SSC 82-20 | ≤ 0.00025 | ≤ 0.000031 | ≤ 0.000031 | ≤ 0.000031 |
| " " | SSC 82-26 | ≤ 0.00025 | ≤ 0.000031 | ≤ 0.000031 | ≤ 0.000031 |
| " " | SSC 82-24 | ≤ 0.00025 | ≤ 0.000031 | ≤ 0.000031 | ≤ 0.000031 |
| " " | SSC 82-57 | ≤ 0.00025 | ≤ 0.000031 | ≤ 0.000031 | ≤ 0.000031 |
| Staphylococcus epidermidis | CMC-83-133 | ≤ 0.00025 | ≤ 0.000031 | ≤ 0.000031 | ≤ 0.000031 |
| " " | ATCC 12228 | ≤ 0.00025 | ≤ 0.000031 | ≤ 0.000031 | ≤ 0.000031 |
| Enterococcus sp. | CMC 83-53 | 0.0038 | 0.031 | 0.062 | 0.0078 |
| Streptococcus faecalis | ATCC 29212 | ≤ 0.00025 | 0.00012 | < 0.000031 | 0.00012 |
| Micrococcus luteus | PCI 1001 | ≤ 0.00025 | ≤ 0.000031 | ≤ 0.000031 | ≤ 0.000031 |
| Bacillus subtilis | ATCC | ≤ 0.00025 | ≤ 0.000031 | ≤ 0.000031 | ≤ 0.000031 |

EP 0 182 152 B1

Certain in vivo testing systems and protocols have been developed by the National Cancer Institute for testing compounds to determine their suitability as anti-neoplastic agents. These have been reported in "Cancer Chemotherapy Reports", Part III, Vol. 3, No. 2 (1972), Geran, et al. These protocols have established standardized screening tests which are generally followed in the field of testing for antitumor agents. Of these systems, lymphocytic leukemia P388, melanotic melanoma B16, L1210 leukemia and colon 26 adenocarcinoma are particularly significant to the present invention. These neoplasms are utilized for testing as transplantable tumors in mice. Generally, significant anti-tumor activity, shown in these protocols by a percentage increase of mean survival times of the treated animals (T) over the control animals (C), is indicative of similar results in human leukemias and solid tumors.

Lymphocytic Leukemia P388 Test

The animals used were $BDF_1$ mice, all of one sex, weighing a minimum of 17 g and all within a 3 g weight range. There were 5 or 6 mice per test group. The tumor transplant was by intraperitoneal injection of 0.5 ml of dilute ascitic fluid containing $10^6$ cells of lymphocytic leukemia P388. LL-E33288 antibiotics were tested in the P388 system both as the individual $\beta_1$-Br and $_1$-Br components and as a complex of all components (Bromo-complex). The test compounds were administered intrapertioneally at a volume of 0.5 ml in 0.2% Klucel in normal saline on days 1, 5 and 9 (relative to tumor inoculation) at the indicated doses. The mice were weighed and the survivors recorded on a regular basis for 30 days. The median survival time and the ratio of survival time for treated (T)/control (C) animals were calculated. The positive control compound was Cisplatin given as an intraperitoneal injection in 0.5 ml of 0.2% Klucel on days 1, 5 and 9 at the indicated doses. The results appear in Table IV.
If T/C X 100 (%) is 125 or over, the tested compound is considered to have significant anti-tumor activity.

TABLE IV

| Lymphocytic Leukemia P388 Test | | | |
|---|---|---|---|
| Compound | Dose (mg/kg) | Median Survival (Days) | T/C x 100 (%) |
| LL-E33288 | 3.2 | 16.5 | 156 |
| (Bromo-complex) | 1.6 | 17.5 | 165 |
| | 0.8 | 18.5 | 175 |
| | 0.4 | 19 | 179 |
| | 0.2 | 16.5 | 156 |
| Control | - | 10.6 | - |
| Positive Control | 1.0 | 21.5 | 203 |
| | 0.25 | 15 | 142 |
| | 0.06 | 14.5 | 137 |
| LL-E-33288$\beta_1$ -Br | 0.4 | 13 | 105 |
| | 0.2 | 18 | 145 |
| | 0.1 | 19 | 153 |
| | 0.05 | 17.5 | 141 |
| | 0.025 | 18 | 145 |
| | 0.012 | 14 | 113 |
| Control | - | 12.4 | - |
| Positive Control | 1.0 | 25.5 | 206 |
| | 0.4 | 19 | 153 |
| | 0.06 | 15 | 121 |
| LL-E33288$\gamma_1$-Br | 0.2 | 14 | 113 |
| | 0.1 | 21 | 169 |
| | 0.05 | 19.5 | 157 |
| | 0.025 | 18 | 145 |
| | 0.012 | 14.5 | 117 |

TABLE IV   (continued)

| Lymphocytic Leukemia P388 Test | | | |
|---|---|---|---|
| Compound | Dose (mg/kg) | Median Survival (Days) | T/C x 100 (%) |
| Control | - | 12.4 | - |
| Positive Control | 1.0 | 25.5 | 206 |
| | 0.4 | 19 | 153 |
| | 0.06 | 15 | 121 |

Melanotic Melanoma B16

The animals used were $BDF_1$ mice, all of the same sex, weighing a minimum of 17 g and all within a 3 g weight range. There are normally 6 animals per test group. A 1 g portion of melanotic melanoma $\beta_{16}$ tumor was homogenized in 10 ml of cold balanced salt solution and a 0.5 ml aliquot of the homogenate was implanted intraperitoneally into each of the test mice. LL-E33288 antibiotics were tested in the $\beta_{16}$ system both as the individual $\beta_1$-Br and $\gamma_1$-Br components and as a complex of all components (Bromo-complex). The test compounds were administered intraperitioneally on days 1 through 9 (relative to tumor inoculation) at various doses. The mice were weighed and survivors recorded on a regular basis for 60 days. The median survival time and the ratio of survival time for treated (T)/control (C) animals were calculated. The positive control compounds were Cisplatin or Adriamycin. The results of this test appear in Table V. If T/C X 100 (%) is 125 or over, the tested compound is considered to have significant anti-tumor activity.

TABLE V

| Melanotic Melanoma $B_{16}$ Test | | | |
|---|---|---|---|
| Compound | Dose (mg/kg) | Median Survival (Days) | T/C x 100 (%) |
| LL-E33288 | 0.8 | 30 | 188 |
| (Bromo-complex) | 0.4 | 29.5 | 184 |
| | 0.2 | 27 | 169 |
| | 0.1 | 24 | 150 |
| Control | - | 16 | - |
| Cisplatin | 0.4 | 25 | 156 |
| | 0.2 | 25 | 156 |
| | 0.1 | 23 | 144 |
| | 0.05 | 21.5 | 134 |
| LL-E33288$\beta_1$-Br | 0.05 | 32 | 168 |
| | 0.025 | 33.5 | 176 |
| | 0.0125 | 32 | 168 |
| Control | - | 19 | - |
| Adriamycin | 0.8 | > 60 | > 316 |
| | 0.4 | > 60 | > 316 |
| LL-E33288$\gamma_1$-Br | 0.05 | 33.5 | 176 |
| | 0.025 | 30 | 159 |
| | 0.0125 | 37 | 195 |

TABLE V   (continued)

| Melanotic Melanoma $B_{16}$ Test | | | |
|---|---|---|---|
| Compound | Dose (mg/kg) | Median Survival (Days) | T/C x 100 (%) |
| Control | - | 19 | - |
| Adriamycin | 0.8 | > 60 | > 316 |
|  | 0.4 | > 60 | > 316 |

Lymphocytic Leukemia L1210 Test

The animals used were $BDF_1$ mice, all of one sex, weighing a minimum of 17 g and all within a 3 g weight range. There were 6 mice in each test group and 18 in control groups. The tumor transplant was by intraperitoneal injection of 0.5 ml of lymphocytic leukemia L1210 at a concentration of $10^5$ cells per mouse. LL-E33288 antibiotics were tested in the L1210 system both as the individual $\beta_1$-Br component and as a complex of all components (Bromo-complex). The test compounds were administered on days 1, 5 and 9 or days 1 through 9 (relative to tumor inoculation) at various doses. The mice were weighed and survivors recorded on a regular basis for 30 days. The median survival time and the ratio of survival time for treated (T)/control (C) animals were calculated. The positive control compound was 1,4-dihydroxy-5,8-bis[[2-(2-hydroxyethylamino)ethyl]amino]anthraquinone dihydrochloride or Cisplatin given intraperitoneally at the indicated dose. The results appear in Table VI. If T/C X 100 (%) is 125 or over, the tested compound is considered to have significant anti-tumor activity.

TABLE VI

| Lymphocytic Leukemia L1210 Test | | | |
|---|---|---|---|
| Compound | Dose (mg/kg) | Median Survival (Days) | T/C x 100 (%) |
| LL-E33288 | 1.5 | 29 | 174 |
| (Bromo-complex) | 0.8 | 28.5 | 171 |
|  | 0.4 | 22.5 | 135 |
|  | 0.2 | 22.5 | 135 |
| Control | - | 16.7 | - |
| Anthraquinone | 1.6 | 30 | 180 |
|  | 0.8 | 30 | 180 |
|  | 0.4 | 30 | 180 |
| LL-E33288$\beta_1$-Br | 0.2 | 11.3 | 136 |
|  | 0.1 | 11.4 | 137 |
|  | 0.05 | 11 | 133 |
|  | 0.025 | 11.3 | 136 |
| Control | - | 8.3 | - |
| Cisplatin | 5 | 7.5 | 90 |
|  | 2.5 | 12 | 145 |
|  | 1.25 | 11 | 133 |

Colon 26 Adenocarcinoma Test

The animals used were $CD_2F_1$ female mice weighing a minimum of 17 g and all within a 3 g weight range. There were 5 or 6 mice per test group with three groups of 5 or 6 animals used as untreated controls for each test. The tumor implant was by intraperitoneal (or subcutaneous) injection of 0.5 ml of a 2% Colon 26 tumor brei in Eagle's

**EP 0 182 152 B1**

MEM medium containing antibiotics. LL-E33288 antibiotics were tested in the Colon 26 system as a complex (Bromo-complex) of all components. The test compounds were administered intraperitoneally on days 1, 5 and 9 (relative to tumor implant doses). The mice were weighed and deaths recorded on a regular basis for 30 days. The median survival times for treated (T)/control (C) animals were calculated. The positive control compound was Cisplatin. The results appear in Table VII. If T/C X 100 (%) is 130 or over, the tested compound is considered to have significant anti-tumor activity.

TABLE VII

| Colon 26 Adenocarcinoma Test | | | |
|---|---|---|---|
| Compound | Dose (mg/kg) | Median Survival (Days) | T/C x 100 (%) |
| LL-E33288 | 1.5 | 39.5 | 212 |
| (Bromo-complex) | 0.8 | 32.5 | 175 |
| | 0.4 | 34 | 183 |
| | 0.2 | 25.5 | 137 |
| Control | - | 18.6 | - |
| Positive | 1 | 29 | 156 |
| Control | 0.5 | 38.5 | 207 |
| | 0.25 | 37.5 | 202 |

M5076 Sarcoma

The M5076 reticular cell Sarcoma is propagated as subcutaneous implants in C57B2/6 female mice. In the assays for antitumor activity, $BDF_1$ mice of either sex were inoculated intraperitoneally with 0.5 ml of a 10% tumor brei. LL-E33288 antibiotics were tested in the M5076 system as a complex (Bromo-complex) of all components. Test compounds were administered intraperitoneally on days 1, 5, 9, 13 and 17 relative to tumor inoculation on day zero. The median survival time in days was determined for each drug dose used on day 60 and the ratio of survival time for treated (T)/control (C) animals were calculated. The results of this test appear in Table VIII compared to the results obtained with Cisplatin. If T/C X 100 (%) is 125 or over, the tested compound is considered to have significant anti-tumor activity.

TABLE VIII

| M5076 Sarcoma | | | |
|---|---|---|---|
| Compound | Dose (mg/kg) | Median Survival (Days) | T/C x 100 (%) |
| LL-E33288 | 1.5 | 50 | 175 |
| (Bromo-complex) | 0.8 | 50 | 175 |
| | 0.4 | 39.5 | 139 |
| Control | - | 28.5 | - |
| Cisplatin | 1 | 30 | 105 |
| | 0.5 | 44.5 | 156 |
| | 0.25 | 45 | 158 |

In the same manner, the following iodo-components were tested for antineoplastic activity.

TABLE IX

| Lymphocytic Leukemia P388 Test | | | |
|---|---|---|---|
| Compound | Dose (mg/kg) | Median Survival (Days) | T/C x 100 (%) |
| LL-E33288$\gamma_1$-I (Test 1) | .005 | >25.5 | >196 |
| | .0025 | 22 | 169 |
| | .00125 | 18.5 | 142 |

18

TABLE IX   (continued)

| Lymphocytic Leukemia P388 Test | | | |
|---|---|---|---|
| Compound | Dose (mg/kg) | Median Survival (Days) | T/C x 100 (%) |
| | .0006 | 18 | 138 |
| | .0003 | 15.5 | 119 |
| | .00015 | 15 | 115 |
| Control | - | 13 | - |
| Positive Control Novantrone®* | 1.6 | 22.5 | 173 |
| LL-E33288 $\gamma_1$-I (Test 2) | .01 | 11 | 100 |
| | .005 | 18 | 164 |
| | .0025 | 22.5 | 205 |
| | - .00125 | 18.5 | 168 |
| | .0006 | 16 | 145 |
| | .0003 | 14 | 127 |
| | .00015 | 14 | 127 |
| Control | - | 11 | - |
| Positive Control Novantrone® | 1.6 | 19 | 173 |
| | 0.8 | 16 | 145 |

*1,4-dihydroxy-5,8-bis[[2-(2-hydroxyethylamino)ethyl] amino]anthraquinone-2HCl

TABLE X

| Melanotic Melanoma $B_{16}$ Test | | | |
|---|---|---|---|
| Compound | Dose (mg/kg) | Median Survival (Days) | T/C x 100 (%) |
| LL-E33288 $\gamma_1$-I | .0025 | 26.5 | 156 |
| | .00125 | 27 | 159 |
| | .0006 | 42.5 | 250 |
| | .0003 | 35.5 | 209 |
| | .00015 | 33.5 | 197 |
| | .00007 | 30.5 | 179 |
| Control | - | | - |
| Adriamycin | 0.8 | 48 | 282 |
| | 0.4 | 40 | 235 |
| | 0.2 | 34.5 | 203 |

TABLE XI

| Lymphocytic Leukemia L1210 Test | | | |
|---|---|---|---|
| Compound | Dose (mg/kg) | Median Survival (Days) | T/C x 100 (%) |
| LL-E33288 $\gamma_1$-I | .01 | 8 | 89 |
| | .005 | 14 | 156 |

TABLE XI   (continued)

| Lymphocytic Leukemia L1210 Test | | | |
|---|---|---|---|
| Compound | Dose (mg/kg) | Median Survival (Days) | T/C x 100 (%) |
| | .0025 | 11 | 122 |
| | .0012 | 10.5 | 117 |
| | .0006 | 10 | 111 |
| Control | - | 9 | - |
| Positive Control Novantrone® | 3.2 | 15 | 167 |
| | 1.6 | 11.5 | 128 |
| | 0.8 | 12 | 133 |
| | 0.4 | 11 | 122 |

TABLE XII

| Colon 26 Adenocarcinoma Test | | | |
|---|---|---|---|
| Compound | Dose (mg/kg) | Median Survival (Days) | T/C x 100 (%) |
| LL-E33288 $Y_1$-I | .01 | 11 | 59 |
| | .005 | 25.5 | 138 |
| | .0025 | 27 | 146 |
| | .00125 | 22.5 | 122 |
| | ,0006 | 23.5 | 127 |
| | .0003 | 20 | 108 |
| | .00015 | 17.5 | 95 |
| | .00007 | 17 | 92 |
| Control | - | 18.5 | - |
| Positive Control Cisplatin | 2 | 15.5 | 84 |
| | 1 | 27.5 | 149 |
| | 0.5 | 23.5 | 127 |

General Fermentation Conditions

Cultivation of Micromonospora echinospora NRRL 15839 or NRRL 15975 may be carried out in a wide variety of liquid culture media. Media which are useful for the production of these novel antibacterial and antitumor agents include an assimilable source of carbon, such as starch, sugar, molasses, glycerol, etc.; an assimilable source of nitrogen such as protein, protein hydrolysate, polypeptides, amino acids, corn steep liquor, etc.; and inorganic anions and cations, such as potassium, sodium, ammonium, calcium, sulfate, carbonate, phosphate, chloride, etc. and sources of either bromine (sodium bromide) or iodine (potassium iodide). Trace elements such as boron, molybdenum, copper, etc., are supplied as impurities of other constituents of the media. Aeration in tanks and bottles is supplied by forcing sterile air through or onto the surface of the fermenting medium. Further agitation in tanks is provided by a mechanical impeller. An antifoam agent such as silicone may be added as needed.

General Procedure for the Isolation and Separation of the Antibiotics - LL-E33288

The LL-E33288 antibiotics are recovered from the fermentation broth by extracting the whole mash with an organic solvent such as ethyl acetate or dichloromethane. The antibiotic complex, contained in the organic extract, is further purified by selective precipitation from lower hydrocarbons. The crude LL-E-33288 antibiotic complex thus obtained is further purified and separated into the individual components by a series of column chromatographies using silica gel, Sephadex® LH-20 (Pharmacia Fine Chemicals) and $C_{18}$ bonded silica.

The invention will be described in greater detail in conjunction with the following non-limiting specific examples.

## Example 1

## Inoculum Preparation

A typical medium used to grow the primary inoculum was prepared according to the following formula:

| Beef extract | about 0.3% |
|---|---|
| Tryptone | about 0.5% |
| Dextrose | about 0.5% |
| Dextrin | about 2.4% |
| Calcium carbonate | about 0.4% |
| Yeast extract | about 0.5% |
| Water qs to | 100% |

This medium was adjusted to pH 7.0 and then sterilized. A 100 ml portion of this sterile medium, in a flask, was inoculated with frozen mycelia of the culture NRRL 15839. The inoculated medium was placed on a rotary shaker and agitated vigorously for 48 hours at 32°C. This incubated medium was then used to inoculate 10 liters of the above sterile medium in a 14 liter fermentor. This medium was incubated, with agitation, at 32°C for 48 hours, providing secondary inoculum. This secondary inoculum was then used to inoculate 300 liters of the above sterile medium in a tank and incubated for 48 hours at 30°C while agitated by an impeller driven at 180-200 rpm, providing the tertiary or seed inoculum.

## Example 2

## Tank Fermentation

A fermentation medium was prepared according to the following formulation:

| Dextrose | about 0.5% |
|---|---|
| Sucrose | about 1.5% |
| Peptone, bacteriological grade | about 0.2% |
| Dibasic potassium phosphate | about 0.01% |
| Molasses | about 0.5% |
| Calcium carbonate | about 0.5% |
| Source of bromine or iodine | trace amounts |
| Water qs to | 100% |

A 2800 liter portion of the above medium was sterilized and then inoculated with 300 liters of tertiary (seed) inoculum prepared as described in Example 1. Aeration was supplied at the rate of 0.53 liters of sterile air per liter of mash per minute and agitation was supplied by an impeller driven at 110 rpm. The temperature was maintained at about 28°C and the fermentation was terminated after about 97 hours, at which time the mash was harvested.

The fermentation was monitored for production of the LL-E33288 antibiotics by antibacterial activity, biochemical induction assay, TLC and HPLC analyses.

The whole harvest mash was adjusted to pH 6 and then extracted with 1/2 mash volume ethyl acetate. The ethyl acetate extract was concentrated to a syrup which was washed twice with hexane and filtered through diatomaceous earth. The diatomaceous earth cake was thoroughly mixed with ethyl acetate and filtered. The filtrate was concentrated to 3 liters, dried over excess anhydrous sodium sulfate and then precipitated by the addition of hexane giving about 26.7 g of crude LL-E33288 complex.

Example 3

Separation of LL-E33288 $\alpha_1$-Br, $\alpha_2$-Br, $\alpha_3$-Br and $\alpha_4$-Br from LL-E33288 $\beta_1$-Br, $\beta_2$-Br and $\gamma_1$-Br

The approximately 26.7 g of crude LL-E33288 complex (Bromo-complex) from Example 2 was divided evenly into three portions and chromatographed on three separate 2.4 x 110 cm silica gel columns (Silica Woelm®, 32-63 m, Woelm Pharma) packed and equilibrated with ethyl acetate saturated with 0.1$\underline{M}$ aqueous potassium dihydrogen phosphate. The columns were first eluted with the same solvent at a flow rate of 3 ml/minute for 18 hours, collecting 18 ml fractions. The eluent was changed to ethyl acetate:methanol (95:5) and elution continued for 8 hours. Finally the columns were eluted with ethyl acetate:methanol (90:10) for 10 hours. The fractions were assayed by the modified biochemical induction assay (BIA). The positive fractions were analysed by TLC using silica gel 60 precoated sheets and developed with the solvent system 3% isopropanol in ethyl acetate saturated with 0.1M aqueous potassium dihydrogen phosphate and detected by bioautography using the modified BIA.

Fractions containing LL-E33288$\alpha_1$-Br,$\alpha_2$-Br, $\alpha_3$-Br and $\alpha_4$-Br (LL-E33288$_\alpha$-Br complex) from the three columns were pooled, concentrated to dryness and the residue was dissolved in ethyl acetate and washed with a small amount of water. The ethyl acetate solution was dried over anhydrous sodium sulfate and precipitated as before to yield about 4.2 g of crude LL-E33288$_\alpha$-Br complex.

Fractions containing LL-E33288$\beta_2$-Br, $\beta_1$-Br and $\gamma_1$-Br (LL-E33288 $\beta$ -Br complex containing $\gamma$ -Br from the three columns were pooled and worked up as above to yield about 2.0 g of crude LL-E33288 $\beta$ -Br complex containing $\gamma$-Br.

Example 4

Isolation of LL-E33288 $\beta_1$ -Br and LL-E33288 $\gamma_1$ -Br

An approximately 1.9 g sample of the LL-E33288$\beta$-Br complex containing $\gamma$-Br from Example 3 was chromatographed on a 25 x 10 cm Sephadex® LH-20 column equilibrated with methanol:water (90:10) at a flow rate of 1.2 ml/minute, collecting 15 ml fractions. The fractions were assayed in the BIA and those active were analysed by TLC as before. Fractions 21-26 containing most of the LL-E33288$\beta_1$-Br, $\beta_2$-Br and $\gamma_1$-Br, were pooled and concentrated to remove methanol and the resulting aqueous mixture was lyophilized to yield about 435 mg of partially purified complex containing approximately 10% of LL-E33288 $\beta_1$-Br, 1% of LL-E33288$\beta_2$-Br and 4% of LL-E33288 $\gamma_1$-Br.

The above partially purified LL-E33288$\beta$-Br complex containing $\gamma$-Br was divided evenly and chromatographed on two 1.5 x 100 cm silica gel columns (Kiesel Gel 60, 40-63$\mu$m, EM Products for chromatography) packed and equilibrated with ethyl acetate:methanol (98.2) at a flow rate of 1 ml/minute, collecting 12 ml fractions. The fractions were assayed and analysed by TLC as before and those containing primarily LL-E33288$\beta_1$-Br were pooled, concentrated and precipitated from hexane to yield about 26 mg of 80% pure LL-E33288$_{\beta 1}$-Br. Those fractions containing LL-E33288$\gamma_1$-Br (chromatographing just after LL-E33288 $\beta_1$ -Br) were pooled and worked up to yield about 4.5 mg of 30% pure LL-E33288$\gamma_1$-Br. A few fractions containing LL-E33288$\beta_2$-Br (chromatographing just before LL-E33288 $\beta_1$-Br), were pooled and worked up to yield a trace amount of LL-E33288$\beta_2$-Br.

Example 5

Final Purification of LL-E33288$\beta_1$ -Br

The approximately 26 mg of 80% pure LL-E33288$\beta_1$-Br from Example 4 was combined with other LL-E33288 $\beta_1$-Br samples of similar purity derived from other fermentations conducted under identical conditions. A total of about 38 mg of this combined $\beta_1$-Br was further purified by reverse phase preparative TLC using Whatman PLKC$_{18}$F, 100 m precoated TLC plates, developed with methanol:0.1$\underline{M}$ ammonium acetate buffer at pH 4.0 (90:10). The band containing LL-E33288$\beta_1$-Br, chromatographing at $R_f$=0.66 and visualized by quenching effect under short wavelength UV lamp (254 nm), was-excised and the antibiotic was washed off the adsorbant with 10% isopropyl alcohol in ethyl acetate saturated with 0.1$\underline{M}$ aqueous potassium dihydrogen phosphate. The solution was concentrated and the residue was dissolved in ethyl acetate and washed with a small amount of water. The organic solution containing LL-E33288 $\beta_1$-Br was worked up as before to yield about 24.5 mg of 90% pure LL-E33288 $\beta_1$-Br. This sample was further purified by preparative TLC on silica gel (Silica Gel GF precoated plates, 1000 m, Analtech) developed with 3% isopropyl alcohol in ethyl acetate saturated with 0.1$\underline{M}$ aqueous potassium dihydrogen phosphate. The major quenching band under short wavelength UV lamp (254 nm), chromatographing at $R_f$=0.7, was excised and the antibiotic was washed off the adsorbant with dichloromethane:methanol (80:20). The organic solution containing LL-E33288$\beta_1$-Br was worked up as before to yield about 18.8 mg of substantially pure LL-E33288 $\beta_1$-Br.

Example 6

Final Purification of LL-E33288$\beta_1$- Br

The approximately 4.5 mg of 30% pure LL-E33288 $\gamma_1$-Br from Example 4 was combined with other LL-E33288 $\gamma_1$-Br samples of similar purity derived from other fermentations conducted under identical conditions. A total of 18 mg of this combined sample was further purified by preparative TLC on silica gel (Silica Gel GF precoated tapered plates, Analtech) developed with 2% isopropyl alcohol in ethyl acetate saturated with 0.1$\underline{M}$ aqueous potassium dihydrogen phosphate. The major quenching band under short wavelength UV lamp (254 nm), chromatographing at $R_f$=0.5, was excised and worked up as before to yield about 4.3 mg of substantially pure LL-E33288 $\gamma_1$-Br.

A preferred fermentation medium for production of LL-E33288 Bromo-complex is as follows:

| Ingredient | Percent |
|---|---|
| Sucrose | 2.0 |
| Ferrous Sulfate Heptahydrate | 0.01 |
| Magnesium Sulfate Heptahydrate | 0.02 |
| Calcium Carbonate | 0.5 |
| Peptone | 0.2 |
| Molasses | 0.5 |
| Sodium Bromide | 0.05 |
| Water         qs         to | 100 |

However, the addition of iodine as potassium iodide, to the fermentation medium provided substantial improvements by:

1) markedly enhancing vegetative growth in the fermentation;

2) increasing zones of inhibition in bioassays versus <u>Escherichia</u> <u>coli</u> #300 and <u>Bacillus</u> <u>subtilis</u> #308;

3) providing substantial activity at the Rf of LL-E33288$\beta_1$-I and $\gamma_1$-I on the bioautography of TLC plates; and

4) enhancement of other components as detected by TLC.

The following two media are preferred for the production of LL-E33288 Iodo-complex:

| Ingredient | Percent | |
|---|---|---|
| | Media A | Media B |
| Sucrose | 2.0 | 2.0 |
| Ferrous Sulfate Heptahydrate | 0.01 | 0.01 |
| Magnesium Sulfate Heptahydrate | 0.02 | 0.02 |
| Calcium Carbonate* | 0.5 | 0.25 |
| Peptone** | 0.2 | 0.2 |
| Molasses | 0.5 | 0.5 |
| Potassium iodide | 0.05 | 0.01 |
| Water qs to | 100 | 100 |

* Mississippi lime.

** Best results were obtained with MARCOR® bacteriological peptone, but other peptones usable and also polypeptides from meat and casein hydrolyzates.

Example 7

A mycelial-spore suspension was prepared by scraping the surface of a slant of culture NRRL-15839 to which 5 ml of sterile distilled water had been added. This suspension was then used to inoculate 100 ml of sterile seed medium of the following formula:

| Yeast Extract | 0.5% |
|---|---|
| Beef Extract | 0.3% |
| Tryptose | 0.5% |
| Starch | 2.4% |
| Dextrose | 0.5% |
| Calcium Carbonate | 0.4% |
| Water        qs        to | 100.0% |

in a 500 ml flask. This seed flask was incubated at 28°C on a rotary shaker at 200 rpm for 3-4 days, producing Stage I inoculum.

The Stage I inoculum was used to inoculate a Stage II inoculum of the same sterile medium, which was incubated under the same conditions for 2 days.

The Stage II inoculum was then used to inoculate 100 ml of sterile fermentation medium of the formula:

| Sucrose | 2.0% |
|---|---|
| Ferrous Sulfate Heptahydrate | 0.01% |
| Magnesium Sulfate Heptahydrate | 0.02% |
| Calcium Carbonate | 0.5% |
| Peptone (MARCOR®) | 0.2% |
| Molasses | 0.5% |
| Potassium Iodide | 0.05% |
| Water        qs        to | 100.0% |

This medium was incubated at 28°C on a shaker at 200 rpm for 5 days at which time the mash was harvested.

A concentration of 4 to 20 $\mu$g/ml of potassium iodide appears to be optimal, but concentrations of 2 mg/ml do not appear to depress yields.

NRRL-15839 can be induced to produce LL-E33288 $\beta_1$-I when potassium iodide is present in the medium, but only at very low levels (0.2-0.3$\mu$g/ml) as against 1.5-3.5 $\mu$g/ml for the better producing NRRL-15975.

Yields of $\beta_1$-I and $\gamma_1$-I in an iodine medium are 2 to 8 times greater than yields of corresponding brominated compounds $\beta_1$-Br and $\gamma_1$-Br in a bromine medium using NRRL-15975.

Example 8

Separation of LL-E33288 $\alpha_1$-I, $\alpha_2$-I, and $\alpha_3$-I from LL-E33288 $\beta_1$-I, $\beta_2$-I, $\gamma_1$-I and $\delta_1$-I

Approximately 41.3 g of crude LL-E33288 complex derived from the processing 7500 liters of a fermentation using NRRL-15975 and medium containing inorganic iodide was divided evenly into two portions and chromatographed on two separate 2.5 x 110 cm silica gel column (Silica Woelm, 32-63 um) packed and equilibrated with ethyl acetate. The columns were first eluted with ethyl acetate at a flow rate of 4 ml/minute for 4 hours, collecting 20 ml fractions. The eluent was changed to a concave gradient from ethyl acetate saturated with 0.1 $\underline{M}$ aqueous potassium dihydrogen phosphate to 10% isopropyl alcohol in ethyl acetate saturated with 0.1 $\underline{M}$ aqueous potassium dihydrogen phosphate over 24 hours. The columns were finally eluted with 10% isopropyl alcohol in ethyl acetate saturated with 0.1 $\underline{M}$ aqueous potassium dihydrogen phosphate over night. The fractions were assayed in the BIA and those active were analysed by TLC as described in Example 3.

Fractions (86-107) containing LL-E33288$\alpha_3$-I from the two columns were pooled and worked up as before to yield about 2.1 g of crude LL-E33288$\alpha_3$-I.

Fractions (182-253) containing LL-E33288$\alpha_1$-I and $\alpha_2$-I from the two columns were pooled and worked up to yield about 4.2 g of a crude mixture of LL-E33288 $\alpha_1$-I and $\alpha_2$-I.

Fractions ( 254-272) containing LL-E33288$\beta_2$-I and $\beta_1$-I from the two columns were pooled and worked up to yield about 1.2 g of a crude mixture of LL-E33288 $\beta_2$-I and $\beta_1$-I.

Fractions (273-302) containing LL-E33288 $\gamma_1$-I from the two columns were pooled and worked up to yield about 1.9 g of 30% pure LL-E33288 $\gamma_1$-I.

Fractions (303-340) containing LL-E33288$\delta_1$-I from the two columns were pooled and worked up to yield about 1.3 g of partially purified LL-E33288 $\delta_1$-I.

Example 9

Purification of LL-E33288 $\gamma_1$-I

Approximately 900 mg of the 30% pure LL-E33288 $\gamma_1$-I from Example 8 was chromatographed on a 2.5 x 120 cm sephadex LH-20 column equilibrated with ethyl acetate:dichloromethane:ethanol (2:2:1) at a flow rate of 1 ml/minute, collecting 12 ml fractions. The fractions were assayed and analysed by TLC as before and those containing LL-E33288 $\gamma_1$-I (fractions 24-33) were pooled and worked up to yield 428 mg of 64% pure LL-E33288 $\gamma_1$-I.

A 22 mg sample of the above was chromatographed on a 0.8 x 24 cm Sepralyte $C_{18}$ (35-60 um, Analytichem) column equilibrated with acetonitrile:0.2 $\underline{M}$ aqueous ammonium acetate (55.45) at a flow rate of 2 ml/minute, collecting 12 ml fractions. The fractions were assayed and analysed by TLC as before and those containing pure LL-E33288 $\gamma_1$-I were pooled and worked up to yield 7.7 mg of pure LL-E33288 $\gamma_1$-I.

Example 10

Purification of LL-E33288 $\beta_1$-I and $\beta_2$-I

Approximately 600 mg of the crude mixture of LL-E33288 $\beta_2$-I and $\beta_1$-I from Example 8 was chromatographed on a 2.5 x 120 cm Sephadex LH-20 column equilibrated with ethyl acetate:dichloromethane:ethanol (2:2:1) at a flow rate of 1 ml/minute, collecting 12 ml fractions. The fractions were assayed and analysed by TLC as before and those containing LL-E33288 $\beta_2$-I and LL-E33288 $\beta_1$-I (fractions 23-31) were pooled and worked up to yield 81 mg of a partially purified mixture of LL-E33288 $\beta_2$-I and $\beta_1$-I.

The sample above was chromatographed on a 1.5 x 90 cm Sephadex LH-20 column equilibrated with hexane:dichloromethane:ethanol (3:1:1) at a flow rate of 0.8 ml/minute, collecting 12 ml fractions. The fractions were assayed and analysed by TLC as before and those containing LL-E33288 $\beta_2$-I (fractions 17-30) and LL-E33288 $\beta_1$-I (fractions 31-38) were pooled separately and worked up to yield 31 mg of partially purified LL-E33288 $\beta_2$-I and 20 mg of 80% pure LL-E33288 $\beta_1$-I.

## Claims

1. A compound LL-E33288 $\alpha_1$-Br, having the following $R_f$ values in the indicated solvent systems on TLC on silica gel sheets:

   a) ethyl acetate saturated with 0.1$\underline{M}$ aqueous potassium dihydrogen phosphate, $R_f$=0.67;
   b) 3 % isopropanol in ethyl acetate saturated with 0.1$\underline{M}$ aqueous potassium dihydrogen phosphate, $R_f$=0.80; and
   c) ethyl acetate:methanol (95:5), $R_f$=0.79,

   producible by aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288 $\alpha_1$-Br by a series of column chromatography and assaying and analyzing the compound LL-E33288 $\alpha_1$-Br by thin layer chromatography.

2. A compound LL-E33288 $\alpha_2$-Br, having the following $R_f$ values in the indicated solvent systems on TLC on silica gel sheets:

   a) ethyl acetate saturated with 0.1$\underline{M}$ aqueous potassium dihydrogen phosphate, $R_f$=0.61;

   b) 3% isopropanol in ethyl acetate saturated with 0.1$\underline{M}$ aqueous potassium dihydrogen phosphate, $R_f$=0.75; and

   c) ethyl acetate:methanol (95:5), $R_f$=0.73;

   producible by aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\alpha_2$-Br by a series of column chromatography and assaying and analyzing the compound LL-E33288$\alpha_2$-Br by thin layer chromatography.

3. A compound LL-E33288$\alpha_3$-Br having the following $R_f$ values in the indicated solvent systems on TLC on silica gel sheets:

a) ethyl acetate saturated with 0.1$\underline{M}$ aqueous potassium dihydrogen phosphate, $R_f$=0.55;

b) 3% isopropanol in ethyl acetate saturated with 0.1$\underline{M}$ aqueous potassium dihydrogen phosphate, $R_f$=0.69; and

c) ethyl acetate:methanol (95:5), $R_f$=0.61;

producible by aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\alpha_3$-Br by a series of column chromatography and assaying and analyzing the compound LL-E33288$\alpha_3$-Br by thin layer chromatography.

4. A compound LL-E33288$\alpha_4$-Br, having the following $R_f$ values in the indicated solvent systems on TLC on silica gel sheets:

a) ethyl acetate saturated with 0.1$\underline{M}$ aqueous potassium dihydrogen phosphate, $R_f$=0.49;

b) 3% isopropanol in ethyl acetate saturated with 0.1$\underline{M}$ aqueous potassium dihydrogen phosphate, $R_f$=0.64; and

c) ethyl acetate:methanol (95:5), $R_f$=0.54,

producible by aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24-32°C for a period of approximately 90-200 hours, harvesting the mash and extracting the antibiotics from the fermentation broth with an organic solvent and

purifying the extracted mixture by selective precipitation from lower hydrocarbons, separating LL-E33288$\alpha_4$-Br by a series of columnchromatography and assaying and analyzing the compound LL-E33288$\alpha_4$-Br by thin layer chromatography.

5. A compound LL-E33288$\beta_1$-Br,

a) having an approximate elemental analysis: C 48.6; H 5.6; N 2.9; S 9.1 and Br 5.5;

b) having a melting point: 146-150°C (dec.);

c) having a specific rotation: $[\alpha]_D^{26}$ = -49±10° (0.1%, ethanol);

d) having ultraviolet absorption spectra as shown in Figure I of the drawings;

e) having an infrared absorption spectrum as shown in Figure II of the drawings;

f) having a proton magnetic resonance spectrum as shown in Figure III of the drawings;

g) having a carbon-13 magnetic resonance spectrum as shown in Figure IV of the drawings with significant peaks at:

| | | | |
|---|---|---|---|
| 17.60(q); | 17.64(q); | 18.9(q); | 19.7(q) |
| 22.4(q); | 22.8(q); | 23.5(q); | 34.3(t); |
| 36.9(t); | 39.2(t/d); | 47.8(d); | 51.7(q); |
| 52.7(q); | 54.6(d); | 56.3(q); | 57.2(q); |
| 57.8(d); | 61.0(q/d); | 61.7(d); | 62.4(t); |
| 66.9(d); | 68.4(d); | 69.1(d); | 69.7(d); |
| 70.2(d); | 71.1(d); | 71.9(d); | 72.1(s); |
| 76.1(d); | 81.0(d); | 83.3(s); | 88.2(s); |
| 97.4(d); | 99.7(d); | 100.8(s); | 102.5(d); |
| 115.1(s); | 123.4(d); | 124.4(d); | 126.5(d); |
| 130.2(s); | 130.8(s); | 144.6(s); | 149.3(s); |
| 149.5(s); | 191.7(s); | 192.4(s); | and |

h) having the following $R_f$ values in the indicated solvent systems on TLC on silica gel sheets:

i) ethyl acetate saturated with 0.1$\underline{M}$ aqueous potassium dihydrogen phosphate, $R_f$=0.24;

ii) 3% isopropanol in ethyl acetate saturated with 0.1$\underline{M}$ aqueous potassium dihydrogen phosphate, $R_f$=0.35;

iii) ethyl acetate:methanol (95:5), $R_f$=0.36.

i) having a molecular weight: 1333/1335, respectively for $^{79}$Br/$^{81}$Br; and

j) having a molecular formula: $C_{54}H_{84}N_3O_{22}S_4Br$.

producible by aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\beta_1$-Br by a series of column chromatography and assaying and analyzing the compound LL-E33288$\beta_1$-Br by thin layer chromatography.

6. A compound LL-E33288$\beta_2$-Br, having the following $R_f$ values in the indicated solvent systems on TLC on silica gel sheets:

a) ethyl acetate saturated with 0.1$\underline{M}$ aqueous potassium dihydrogen phosphate, $R_f$=0.32;

b) 3% isopropanol in ethyl acetate saturated with 0.1$\underline{M}$ aqueous potassium dihydrogen phosphate, $R_f$=0.41; and

c) ethyl acetate:methanol (95:5), $R_f$=0.45,

producible by aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24-32°C for a period of approximately 90-200 hours, harvesting the mash and extracting the antibiotics from the fermentation broth with an organic solvent and

purifying the extracted mixture by selective precipitation from lower hydrocarbons, separating LL-E33288$\beta_2$-Br by a series of column chromatography and assaying and analyzing the compound LL-E33288$\beta_2$-Br by thin layer chromatography.

**7.** A compound LL-E33288$\gamma_1$-Br

a) having ultraviolet absorption spectra as shown in Figure V of the drawings;

b) having an infrared absorption spectrum as shown in Figure VI of the drawings;

c) having a proton magnetic resonance spectrum as shown in Figure VII of the drawings; and

d) having the following $R_f$ values in the indicated solvent systems on TLC on silica gel sheets:

i) ethyl acetate saturated with 0.1$\underline{M}$ aqueous potassium dihydrogen phosphate, $R_f$=0.18;

ii) 3% isopropanol in ethyl acetate saturated with 0.1$\underline{M}$ aqueous potassium dihydrogen phosphate, $R_f$=0.28;

iii) ethyl acetate:methanol (95:5), $R_f$=0.27.

e) having a carbon-13 magnetic resonance spectrum as shown in Figure VIII of the drawings with significant peaks at:

| | | | |
|---|---|---|---|
| 14.4 | 17.6 | 17.9 | 19.0 |
| 19.7 | - | 22.8 | - |
| - | 34.0 | 37.5 | 39.5 |
| 42.1 | - | 51.6 | 52.7 |
| 54.1 | 56.3 | 57.3 | - |
| 59.3 | 61.1 | 61.8 | 61.9 |
| 67.2 | 68.18 | 68.23 | 69.7 |
| 70.1 | 70.8 | 71.1 | 71.7 |
| 71.8 | 76.1 | - | 81.0 |
| 82.9 | 88.4 | - | 97.8 |
| 100.0 | 100.2 | 101.3 | 103.0 |
| 115.3 | 123.0 | 124.9 | 126.9 |
| 130.4 | 131.1 | 131.8 | 138.0 |
| 144.7 | - | 149.5 | 149.6 |
| 155.6 | 192.5 | 192.9 | |

f) having a molecular formula: $C_{55}H_{74}N_3O_{21}S_4Br$; and

g) having a molecular weight: 1319/1321, respectively for $^{79}Br/^{81}Br$,

producible by aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\gamma_1$-Br by a series of column chromatography and assaying and analyzing the compound LL-E33288$\gamma_1$-Br by thin layer chromatography.

**8.** A compound LL-E33288$\alpha_1$-I

a) having the following $R_f$ values in the indicated solvent systems on TLC on silica gel sheets:

i) ethyl acetate saturated with 0.1$\underline{M}$ aqueous potassium dihydrogen phosphate, $R_f$=0.67;

ii) 3% isopropanol in ethyl acetate saturated with 0.1$\underline{M}$ aqueous potassium dihydrogen phosphate, $R_f$=0.80; and

iii) ethyl acetate:methanol (95:5), $R_f$=0.80; and

b) having a molecular weight:1145, producible by aerobically fermenting a liquid medium containing assimilable sources of carbon, nitrogen, iodine and inorganic salts, which medium has been inoculated with a viable culture of the microorganism <u>Micromonospora echinospora</u> ssp. <u>calichensis</u> NRRL 15839 or mutants thereof including NRRL 15975, maintaining said fermentation culture at a temperature of about 24-32°C for a period of approximately 90-200 hours, harvesting the mash and extracting the antibiotics from the fermentation broth with an organic solvent and

purifying the extracted mixture by selective precipitation from lower hydrocarbons,
separating LL-E33288$\alpha_1$-I by a series of column chromatography and
assaying and analyzing the compound LL-E33288$\alpha_1$-I by thin layer chromatography.

**9.** A compound LL-E33288$\alpha_2$-I

a) having the following $R_f$ values in the indicated solvent systems on TLC on silica gel sheets:

i) ethyl acetate saturated with 0.1<u>M</u> aqueous potassium dihydrogen phosphate, $R_f$=0.61;

ii) 3% isopropanol in ethyl acetate saturated with 0.1<u>M</u> aqueous potassium dihydrogen phosphate, $R_f$=0.75; and

iii) ethyl acetate:methanol (95:5), $R_f$=0.73;

b) containing only the following elements: C, H, N, O, S and I;

c) having a molecular weight:1131; and

d) having a proton magnetic resonance spectrum as shown in Figure IX of the drawings,

producible by aerobically fermenting a liquid medium containing assimilable sources of carbon, nitrogen, iodine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\alpha_2$-I by a series of column chromatography and assaying and analyzing the compound LL-E33288$\alpha_2$-I by thin layer chromatography.

**10.** A compound IL-E33288$\alpha_3$-I

a) having the following $R_f$ values in the indicated solvent systems on TLC on silica gel sheets:

i) ethyl acetate saturated with 0.1<u>M</u> aqueous potassium dihydrogen phosphate, $R_f$=0.55;

ii) 3% isopropanol in ethyl acetate saturated with 0.1<u>M</u> aqueous potassium dihydrogen phosphate, $R_f$=0.69; and

iii) ethyl acetate:methanol (95:5), $R_f$=0.61;

b) having a molecular weight:1066; and

c) having a proton magnetic resonance spectrum as shown in Figure x of the drawings,

producible by aerobically fermenting a liquid medium containing assimilable sources of carbon, nitrogen, iodine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\alpha_3$-I by a series of column chromatography and assaying and analyzing the compound LL-E33288$\alpha_3$-I by thin layer chromatography.

11. A compound LL-E33288$\beta_1$-I

   a) having the following $R_f$ values in the indicated solvent systems on TLC on silica gel sheets:

   i) ethyl acetate saturated with 0.1$\underline{M}$ aqueous potassium dihydrogen phosphate, $R_f$=0.24;

   ii) 3% isopropanol in ethyl acetate saturated with 0.1$\underline{M}$ aqueous potassium dihydrogen phosphate, $R_f$=0.35; and

   iii) ethyl acetate:methanol (95:5), $R_f$=0.36;

   b) having an ultraviolet absorption spectra as shown in Figure XI of the drawings;

   c) having an infrared absorption spectrum as shown in Figure XII of the drawings;

   d) having a proton magnetic resonance spectrum as shown in Figure XIII of the drawings;

   e) having a carbon 13 magnetic resonance spectrum as shown in Figure XIV of the drawings with significant peaks at

| - | 17.5 | 17.6 | 18.9 |
|---|---|---|---|
| - | 22.4 | 22.8 | 23.4 |
| 25.4 | 34.3 | 36.9 | 39.2 |
| - | 47.9 | 51.6 | 52.8 |
| 54.8 | 56.3 | 57.2 | 57.9 |
| 60.9 | | 61.6 | 62.2 |
| 67.0 | 68.4 | 68.4 | 69.1 |
| 69.6 | 70.4 | 71.1 | 71.8 |
| 72.2 | 76.2 | - | 80.8 |
| 83.3 | 88.1 | 93.6 | 97.4 |
| 99.6 | 99.6 | - | 102.6 |
| 112.4 | 123.4 | 124.4 | 126.4 |
| - | - | 133.4 | - |
| - | - | - | - |
| - | 192.3 | 192.6 | |

   f) having a molecular formula: $C_{54}H_{84}N_3O_{22}S_4I$; and

   g) having a molecular weight:1381, producible by

   aerobically fermenting a liquid medium containing assimilable sources of carbon, nitrogen, iodine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\beta_1$-I by a series of column chromatography and assaying and analyzing the compound LL-E33288$\beta_1$- I by thin layer chromatography.

12. A compound LL-E33288$\beta_2$-I having the following $R_f$ values in the indicated solvent systems on TLC on silica gel sheets:

   a) ethyl acetate saturated with 0.1$\underline{M}$ aqueous potassium dihydrogen phosphate, $R_f$=0.32.

b) 3% isopropanol in ethyl acetate saturated with 0.1$\underline{M}$ aqueous potassium dihydrogen phosphate, $R_f$=0.41; and

c) ethyl acetate:methanol (95:5), $R_f$=0.45,

producible by aerobically fermenting a liquid medium containing assimilable sources of carbon, nitrogen. iodine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24-32°C for a period of approximately 90-200 hours, harvesting the mash and extracting the antibiotics from the fermentation broth with an organic solvent and

purifying the extracted mixture by selective precipitation from lover hydrocarbons,
separating LL-E33288$\beta_2$-I by a series of column chromatography and
assaying and analyzing the compound LL-E33288$\beta_2$-I by thin layer chromatography.

**13.** A compound LL-E33288Y1-I

a) having the following $R_f$ values in the indicated solvent systems on TLC on silica gel sheets:

i) ethyl acetate saturated with 0.1$\underline{M}$ aqueous potassium dihydrogen phosphate, $R_f = 0.18$;
ii) 3% isopropanol in ethyl acetate saturated with 0.1$\underline{M}$ aqueous potassium dihydrogen phosphate, $R_f = 0.28$; and
iii) ethyl acetate: methanol (95:5), Rf = 0.27;

b) containing only the following elements: C, H, N, O, S and I;
c) having an approximate elemental analysis: C 48.8; H 5.4; N 2.8; S 9.0; and I 9.2;
d) having a molecular weight: 1367;
e) having a molecular formula: $C_{53} H_{82} N_3 O_{22} S_4 I$;
f) having an ultraviolet absorption spectra as shown in Figure XV of the drawings;
g) having an infrared absorption spectrum as shown in Figure XVI of the drawings;
h) having a proton magnetic resonance spectrum as shown in Figure XVII of the drawings; and
i) having a carbon 13 magnetic resonance spectrum as shown in Figure XVIII of the drawings, significant peaks as listed below:

| | | | |
|---|---|---|---|
| 14.5(q) | 17.6(q) | 17.6(q) | 18.9(q) |
| - | - | 22.8(q) | - |
| 25.4(q) | 34.1(t) | 37.0(t) | 39.1(t) |
| 42.3(t/s) | - | 51.5(d) | 52.8(q) |
| 54.8(t) | 56.3(q) | 57.2(q) | - |
| 60.4(d) | 60.9(q) | 61.3(t) | 61.7(q) |
| 67.0(d) | 68.4(d) | 68.5(d) | 69.2(d) |
| 69.7(d) | 70.5(d) | 71.1(d) | 71.8(d) |
| 72.1(s) | 75.7(d) | 75.8(d) | 80.9(d) |
| 82.8(s) | 88.1(s) | 93.5(s) | 97.3(d) |
| 99.6d | 99.7(d) | 100.8(s) | 102.6(d) |
| - | 123.4(d) | 124.4(d) | 126.2(d) |
| 130.2(s) | 131.0(s) | 133.4(s) | 139.1(s) |
| 143.0(s) | 145.1 | 150.6(s) | 151.5(s) |
| 154.5 | 192.0(s) | 192.5(s), | |

producible by aerobically fermenting a liquid medium containing assimilable sources of carbon, nitrogen, iodine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200 hours, harvesting the mash and extracting the antibiotics from the fermentation broth with an organic solvent and purifying the extracted mixture by selective precipitation from lower hydrocarbons, separating LL-E33288$\gamma_1$-I by a series of column chromatography and assaying and analyzing the compound LL-E33288$\gamma_1$-I by thin layer chromatography.

14. A compound LL-E33288$\delta_1$-I having the following $R_f$ values in the indicated solvent systems on TLC on silica gel sheets:

a) ethyl acetate saturated with 0.1<u>M</u> aqueous potassium dihydrogen phosphate, $R_f$ = 0.11; and
b) 3% isopropanol in ethyl acetate saturated with 0.1<u>M</u> aqueous potassium dihydrogen phosphate, $R_f$ = 0.19,

producible by aerobically fermenting a liquid medium containing assimilable sources of carbon, nitrogen, iodine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24 to 32°C for a period of approximately 90 to 200. hours, harvesting the mash and extracting the antibiotics from the fermentation broth with an organic solvent and purifying the extracted mixture by selective precipitation from lower hydrocarbons, separating LL-E33288$\delta_1$-I by a series of column chromatography and assaying and analyzing the compound LL-E33288$\delta_1$-I by thin layer chromatography.

15. Use of a compound selected from the group consisting of LL-E33288$\alpha_1$-Br; LL-E33288$\alpha_1$-I; LL-E33288$\alpha_2$-Br; LL-E33288$\alpha_2$-I; LL-E33288$\alpha_3$-Br; LL-E33288$\alpha_3$-I; LL-E33288$\alpha_4$-Br; LL-E33288$\beta_1$-Br; LL-E33288$\beta_1$-I; LL-E33288$\beta_2$-Br; LL-E33288$\beta_2$-I; LL-E33288$\gamma_1$-Br; LL-E33288$\gamma_1$-I; and LL-E33288$\delta_1$-I for the manufacture of medicaments for treating bacterial infections in warmblooded animals.

16. Use of a compound selected from the group consisting of LL-E33288$\alpha_1$-Br; LL-E33288$\alpha_1$-I; LL-E33288$\alpha_2$-Br; LL-E33288$\alpha_2$-I; LL-E33288$\alpha_3$-Br; LL-E33288$\alpha_3$-I; LL-E33288$\alpha_4$-Br; LL-E33288$\beta_1$-Br; LL-E33288$\beta_1$-I; LL-E33288$\beta_2$-Br; LL-E33288$\beta_2$-I; LL-E33288$\gamma_1$-Br; LL-E33288$\gamma_1$-I; and LL-E33288$\delta_1$-I for the manufacture of medicaments for inhibiting the growth of tumors in a mammal.

17. Use of a compound selected from the group consisting of LL-E33288$\alpha_1$-Br; LL-E33288$\alpha_1$-I; LL-E33288$\alpha_2$-Br; LL-E33288$\alpha_2$-I; LL-E33288$\alpha_3$-Br; LL-E33288$\alpha_3$-I; LL-E33288$\alpha_4$-Br; LL-E33288$\beta_1$-Br; LL-E33288$\beta_1$-I; LL-E33288$\beta_2$-Br; LL-E33288$\beta_2$-I; LL-E33288$\gamma_1$-Br; LL-E33288$\gamma_1$-I; and LL-E33288$\delta_1$-I for the manufacture of medicaments for regressing leukemia in a mammal.

18. A process for producing antibiotics LL-E33288$\alpha_1$-Br; LL-E33288$\alpha_2$-Br; LL-E33288$\alpha_3$-Br; LL-E33288$\alpha_4$-Br; LL-E33288$\beta_1$-Br; LL-E33288$\beta_2$-Br and LL-E33288$\gamma_1$-Br which comprises aerobically fermenting the organism <u>Micromonospora echinospora</u> ssp. <u>calichensis</u> NRRL 15839 or mutants thereof including NRRL 15975 in a liquid medium containing assimilable sources of carbon, nitrogen, bromine and inorganic salts, until substantial antibiotic activity is imparted to said medium and then recovering the antibiotics therefrom.

19. A process for producing antibiotics LL-E33288$\alpha_1$-Br; LL-E33288$\alpha_2$-Br; LL-E33288$\alpha_3$-Br; LL-E33288$\alpha_4$-Br; LL-E33288$\beta_1$-Br; LL-E33288$\beta_2$-Br; and LL-E33288$\gamma_1$-Br which comprises aerobically fermenting a liquid medium containing assimilable sources of carbon, nitrogen, bromine and inorganic salts; which medium has been inoculated with a viable culture of the organism <u>Micromonospora echinospora</u> ssp. <u>calichensis</u> NRRL 15839 or mutants thereof including NRRL 15975, maintaining said fermentation culture at a temperature of about 24-32°C for a period of approximately 90-200 hours, harvesting the mash and extracting the antibiotics.

20. A process for producing antibiotics LL-E33288$\alpha_1$-I; LL-E33288$\alpha_2$-I; LL-E33288$\alpha_3$-I; LL-E33288$\beta_1$-I; LL-E33288$\beta_2$-I; LL-E33288$\gamma_1$-I; and LL-E33288$\delta_1$-I which comprises aerobically fermenting the organism <u>Micromonospora echniospora</u> ssp. <u>calichensis</u> NRRL 15839 or mutants thereof including NRRL 15975 in a liquid medium containing assimilable sources of carbon, nitrogen, iodine and inorganic salts, until substantial antibiotic activity is imparted to said medium and then recovering the antibiotics therefrom.

21. A process for producing antibiotics LL-E33288$\alpha_1$-I; LL-E33288$\alpha_2$-I; LL-E33288$\alpha_3$-I; LL-E33288$\beta_1$-I; LL-E33288$\beta_2$-I; LL-E33288$\gamma_1$-I; and LL-E33288$\delta_1$-I which comprises aerobically fermenting a liquid medium containing assimilable sources of carbon, nitrogen, iodine and inorganic salts, which medium has been inoculated with a viable culture of the microorganism <u>Micromonospora echinospora</u> ssp. <u>calichensis</u> NRRL 15839 or mutants thereof including NRRL 15975, maintaining said fermentation culture at a temperature of about 24-32°C for a period of approximately 90-200 hours, harvesting the mash and extracting the antibiotics.

EP 0 182 152 B1

**22.** A culture containing the microorganism <u>Micromonospora echinospora</u> ssp. <u>calichensis,</u> NRRL 15839, said culture being capable of producing the LL-E33288 complex in recoverable quantity upon aerobic fermentation in an aqueous medium containing assimilable sources of carbon nitrogen, inorganic salts, and either iodine or bromine or both.

**23.** A culture containing the microorganism <u>Micromonospora echinospora</u> ssp. <u>calichensis</u> NRRL 15975, said culture being capable of producing the LL-E33288 complex in recoverable quantity upon aerobic fermentation in an aqueous medium containing assimilable sources of carbon, nitrogen, inorganic salts, and either iodine or bromine or both.

**Patentansprüche**

**1.** Verbindung LL-E33288$\alpha_1$ -Br mit den folgenden $R_f$-Werten im angegebenen Lösungsmittelsystem bei Dünnschichtchromatographie auf Silikagel-Platten:

a) Ethylacetat, gesättigt mit 0,1M wässerigem Kaliumdihydrogenphosphat, $R_f$=0,67;
b) 3% Isopropanol in Ethylacetat, gesättigt mit 0,1M wässerigem Kaliumdihydrogenphosphat, $R_f$=0,80; und
c) Ethylacetat:Methanol (95:5), $R_f$=0,79,

herstellbar durch aerobe Fermentation eines flüssigen Mediums, das assimilierbare Quellen für Kohlenstoff, Stickstoff, Brom und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\alpha_1$ -Br durch eine Reihe von Säulenchromatographien und Untersuchen und Analysieren der Verbindung LL-E33288$\alpha_1$ -Br mittels Dünnschichtchromatographie.

**2.** Verbindung LL-E33288$\alpha_2$-Br mit den folgenden $R_f$-Werten in den angegebenen Lösungsmittelsystemen bei Dünnschichtchromatographie auf Silikagel-Platten:

a) Ethylacetat, gesättigt mit 0,1M wässerigem Kaliumdihydrogenphosphat, $R_f$=0,61;
b) 3% Isopropanol in Ethylacetat, gesättigt mit 0,1M wässerigem Kaliumdihydrogenphosphat, $R_f$=0,75; und
c) Ethylacetat:Methanol (95:5), $R_f$=0,73;

herstellbar durch aerobe Fermentation eines flüssigen Mediums, das assimilierbare Quellen für Kohlenstoff, Stickstoff, Brom und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\alpha_2$-Br durch eine Reihe von Säulenchromatographien und Untersuchen und Analysieren der Verbindung LL-E33288$\alpha_2$ -Br mittels Dünnschichtchromatographie.

**3.** Verbindung LL-E33288$\alpha_3$ -Br mit den folgenden $R_f$-Werten in den angegebenen Lösungsmittelsystemen bei Dünnschichtchromatographie auf Silikagel-Platten:

a) Ethylacetat, gesättigt mit 0,1M wässerigem Kaliumdihydrogenphosphat, $R_f$=0,55;
b) 3% Isopropanol in Ethylacetat, gesättigt mit 0,1M wässerigem Kaliumdihydrogenphosphat, $R_f$=0,69; und
c) Ethylacetat:Methanol (95:5), $R_f$=0,61;

herstellbar durch aerobe Fermentation eines flüssigen Mediums, das assimilierbare Quellen für Kohlenstoff, Stickstoff, Brom und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\alpha_3$ -Br durch eine Reihe von Säulenchromatographien und Untersuchen und Analysieren der Verbindung LL-E33288$\alpha_3$ -Br mittels Dünnschichtchromatographie.

**4.** Verbindung LL-E33288$\alpha_4$ -Br mit den folgenden $R_f$-Werten in den angegebenen Lösungsmittelsystemen bei Dünnschichtchromatographie auf Silikagel-Platten;

    a) Ethylacetat, gesättigt mit 0,1M wässerigem Kaliumdihydrogenphosphat, $R_f$=0,49;
    b) 3% Isopropanol in Ethylacetat, gesättigt mit 0,1M wässerigem Kaliumdihydrogenphosphat, $R_f$=0,64; und
    c) Ethylacetat:Methanol (95:5), $R_f$=0,54;

herstellbar durch aerobe Fermentation eines flüssigen Mediums, das assimilierbare Quellen für Kohlenstoff, Stickstoff, Brom und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\alpha_4$-Br durch eine Reihe von Säulenchromatographien und Untersuchen und Analysieren der Verbindung LL-E33288$\alpha_4$ -Br mittels Dünnschichtchromatographie.

**5.** Verbindung LL-E33288$\beta_1$ -Br

    a) mit einer annähernden Elementaranalyse:
    C 48,6; H 5,6; N 2,9; S 9,1 und Br 5,5;
    b) mit einem Schmelzpunkt: 146-150°C (Zers.);
    c) mit einer spezifischen Drehung: $[\alpha]^{26}_D$=-49±10° (0,1%, Ethanol);
    d) mit Ultraviolett-Absorptionsspektren wie in Fig. I der Zeichnungen gezeigt;
    e) mit einem Infrarotspektrum, wie in Fig. II der Zeichnungen gezeigt;
    f) mit einem Protonen-Magnetresonanzspektrum, wie in Fig. III der Zeichnungen gezeigt;
    g) mit einem Kohlenstoff-13-Magnetresonanzspektrum, wie in Fig. IV der Zeichnungen gezeigt, mit signifikanten Peaks bei:

| | | | |
|---|---|---|---|
| 17,60(q); | 17,64(q); | 18,9(q); | 19,7(q) |
| 22,4(q); | 22,8(q); | 23,5(q); | 34,3(t); |
| 36,9(t); | 39,2(t/d); | 47,8(d); | 51,7(q); |
| 52,7(q); | 54,6(d); | 56,3(q); | 57,2(q); |
| 57,8(d); | 61,0(q/d); | 61,7(d); | 62,4(t); |
| 66,9(d); | 68,4(d); | 69,1(d); | 69,7(d); |
| 70,2(d); | 71,1(d); | 71,9(d); | 72,1(s); |
| 76,1(d); | 81,0(d); | 83,3(s); | 88,2(s); |
| 97,4(d); | 99,7(d); | 100,8(s); | 102,5(d); |
| 115,1(s); | 123,4(d); | 124,4(d); | 126,5(d); |
| 130,2(s); | 130,8(s); | 144,6(s); | 149,3(s); |
| 149,5(s); | 191,7(s); | 192,4(s); | und |

    h) mit den folgenden $R_f$-Werten in den angegebenen Lösungsmittelsystemen bei Dünnschichtchromatographie auf Silikagel-Platten:

        i) Ethylacetat, gesättigt mit 0,1M wässerigem Kaliumdihydrogenphosphat, $R_f$=0,24;
        ii) 3% Isopropanol in Ethylacetat, gesättigt mit 0,1M wässerigem Kaliumdihydrogenphosphat, $R_f$=0,35; und
        iii) Ethylacetat:Methanol (95:5), $R_f$=0,36;

    i) mit einem Molekulargewicht: 1333/1335, für $^{79}$Br bzw. $^{81}$Br; und
    j) mit einer Molekülformel: $C_{54} H_{94} N_3 O_{22} S_4$ Br,

herstellbar durch aerobe Fermentation eines flüssigen Mediums, das assimilierbare Quellen für Kohlenstoff, Stickstoff, Brom und anorganische Salze enthält; welches Medium mit einer lebensfähigen Kultur des Organismus <u>Micromonospora</u> <u>echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\beta_1$-Br durch eine Reihe von Säulenchromatographien und Untersuchen und Analysieren der Verbindung LL-E33288$\beta_1$-Br mittels Dünnschichtchromatographie.

6. Verbindung LL-E33288$\beta_2$ mit den folgenden $R_f$-Werten in den angegebenen Lösungsmittelsystemen bei Dünnschichtchromatographie auf Silikagel-Platten:

a) Ethylacetat, gesättigt mit 0,1M wässerigem Kaliumdihydrogenphosphat, $R_f$=0,32;
b) 3% Isopropanol in Ethylacetat, gesättigt mit 0,1M wässerigem Kaliumdihydrogenphosphat, $R_f$=0,41; und
c) Ethylacetat:Methanol (95:5), $R_f$=0,45;

herstellbar durch aerobe Fermentation eines flüssigen Mediums, das assimilierbare Quellen für Kohlenstoff, Stickstoff, Brom und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\beta_2$-Br durch eine Reihe von Säulenchromatographien und Untersuchen und Analysieren der Verbindung LL-E33288$\beta_2$-Br mittels Dünnschichtchromatographie.

7. Verbindung LL-E33288$\gamma_1$-Br

a) mit Ultraviolett-Absorptionsspektren wie in Fig. V der Zeichnungen gezeigt;
b) mit einem Infrarot-Absorptionsspektrum, wie in Fig. VI der Zeichnungen gezeigt;
c) mit einem Protonen-Magnetresonanzspektrum, wie in Fig, VII der Zeichnungen gezeigt; und
d) mit den folgenden $R_f$-Werten in den angegebenen Lösungsmittelsystemen bei Dünnschichtchromatographie auf Silikagel-Platten:

i) Ethylacetat, gesättigt mit 0,1M wässerigem Kaliumdihydrogenphosphat, $R_f$=0,18;
ii) 3% Isopropanol in Ethylacetat, gesättigt mit 0,1M wässerigem Kaliumdihydrogenphosphat, $R_f$=0,28; und
iii) Ethylacetat:Methanol (95:5), $R_f$=0,27;

e) mit einem Kohlenstoff-13-Magnetresonanzspektrum, wie in Fig. VIII der Zeichnungen gezeigt, mit signifikanten Peaks bei:

| | | | |
|---|---|---|---|
| 14,4 | 17,6 | 17,9 | 19,0 |
| 19,7 | - | 22,8 | - |
| - | 34,0 | 37,6 | 39,5 |
| 42,1 | - | 51,6 | 52,7 |
| 54,1 | 56,3 | 57,3 | - |
| 59,3 | 61,1 | 61,8 | 61,9 |
| 67,2 | 68,18 | 68,23 | 69,7 |
| 70,1 | 70,8 | 71,1 | 71,7 |
| 71,8 | 76,1 | - | 81,0 |
| 82,9 | 88,4 | - | 97,8 |
| 100,0 | 100,2 | 101,3 | 103,0 |
| 115,3 | 123,0 | 124,9 | 126,9 |
| 130,4 | 131,1 | 131,8 | 138,0 |
| 144,7 | - | 149,5 | 149,6 |
| 155,6 | 192,5 | 192,9 | |

f) mit einer Molekülformel: $C_{55} H_{74} N_3 O_{21} S_4 Br$; und
g) mit einem Molekulargewicht 1319/1321 für $^{79}$Br bzw. $^{81}$Br, herstellbar durch aerobe Fermentation eines flüssigen Mediums, das assimilierbare Quellen für Kohlenstoff, Stickstoff, Brom und anorganische Salze enthält; welches Medium mit einer lebensfähigen Kultur des Organismus <u>Micromonospora echinospora</u> ssp, <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\gamma_1$-Br durch eine Reihe von Säulenchromatographien und Untersuchen und Analysieren der Verbindung LL-E33288$\gamma_1$ -Br mittels Dünnschichtchromatographie.

**8.** Verbindung LL-E33288$\alpha_1$ -I

a) mit den folgenden $R_f$-Werten in den angegebenen Lösungsmittelsystemen bei Dünnschichtchromatographie auf Silikagel-Platten:

i) Ethylacetat, gesättigt mit 0,1M wässerigem Kaliumdihydrogenphosphat, $R_f$=0,67;
ii) 3% Isopropanol in Ethylacetat, gesättigt mit 0,1M wässerigem Kaliumdihydrogenphosphat, $R_f$=0,80; und
iii) Ethylacetat:Methanol (95:5), $R_f$=0,80; und b) mit einem Molekulargewicht: 1145, herstellbar durch aerobe Fermentation eines flüssigen Mediums, das assimilierbare Quellen für Kohlenstoff, Stickstoff, Jod und anorganische Salze enthält; welches Medium mit einer lebensfähigen Kultur des Mikroorganismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\alpha_1$ -I durch eine Reihe von Säulenchromatographien und Untersuchen und Analysieren der Verbindung LL-E33288$\alpha_1$-I mittels Dünnschichtchromatographie.

**9.** Verbindung LL-E33288$\alpha_2$ -I

a) mit den folgenden $R_f$-Werten in den angegebenen Lösungsmittelsystemen bei Dünnschichtchromatographie auf Silikagel-Platten:

i) Ethylacetat, gesättigt mit 0,1M wässerigem Kaliumdihydrogenphosphat, $R_f$=0,61;
ii) 3% Isopropanol in Ethylacetat, gesättigt mit 0,1M wässerigem Kaliumdihydrogenphosphat, $R_f$=0,75; und
iii) Ethylacetat:Methanol (95:5), $R_f$=0,73;

b) welches nur die folgenden Elemente enthält: C, H, N, O, S und I;
c) mit einem Molekulargewicht: 1131; und
d) mit einem Protonen-Magnetresonanzspektrum, wie in Fig. IX der Zeichnungen gezeigt,

herstellbar durch aerobe Fermentation eines flüssigen Mediums, das assimilierbare Quellen für Kohlenstoff, Stickstoff, Jod und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\alpha_2$-I durch eine Reihe von Säulenchromatographien und Untersuchen und Analysieren der Verbindung LL-E33288$\alpha_2$-I mittels Dünnschichtchromatographie.

**10.** Verbindung LL-E33288$\alpha_3$ -I

a) mit den folgenden $R_f$-Werten in den angegebenen Lösungsmittelsystemen bei Dünnschichtchromatographie auf Silikagel-Platten:

i) Ethylacetat, gesättigt mit 0,1M wässerigem Kaliumdihydrogenphosphat, $R_f$=0,55;
ii) 3% Isopropanol in Ethylacetat, gesättigt mit 0,1M wässerigem Kaliumdihydrogenphosphat, $R_f$=0,69; und
iii) Ethylacetat:Methanol (95:5), $R_f$=0,61;

b) mit einem Molekulargewicht: 1066; und

c) mit einem Protonen-Magnetresonanzspektrum, wie in Fig. X der Zeichnungen gezeigt,

herstellbar durch aerobe Fermentation eines flüssigen Mediums, das assimilierbare Quellen für Kohlenstoff, Stickstoff, Jod und anorganische Salze enthält; welches Medium mit einer lebensfähigen Kultur des Organismus <u>Micromonospora echinosdora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\alpha_3$-I durch eine Reihe von Säulenchromatographien und Untersuchen und Analysieren der Verbindung LL-E33288$\alpha_3$-I mittels Dünnschichtchromatographie.

**11.** Verbindung LL-E33288$\beta_1$-I

a) mit den folgenden $R_f$-werten in den angegebenen Lösungsmittelsystemen bei Dünnschichtchromatographie auf Silikagel-Platten:

i) Ethylacetat, gesättigt mit 0,1M wässerigem Kaliumdihydrogenphosphat, $R_f$=0,24;

ii) 3% Isopropanol in Ethylacetat, gesättigt mit 0,1M wässerigem Kaliumdihydrogenphosphat, $R_f$=0,35; und

iii) Ethylacetat:Methanol (95:5), $R_f$=0,36;

b) mit einem Ultraviolett-Absorptionsspektrum wie in Fig. XI der Zeichnungen gezeigt;

c) mit einem Infrarot-Absorptionsspektrum, wie in Fig. XII der Zeichnungen gezeigt;

d) mit einem Protonen-Magnetresonanzspektrum, wie in Fig. XIII der Zeichnungen gezeigt;

e) mit einem Kohlenstoff-13-Magnetresonanzspektrum, wie in Fig. XIV der Zeichnungen gezeigt, mit signifikanten Peaks bei:

| - | 17,5 | 17,6 | 18,9 |
|---|---|---|---|
| - | 22,4 | 22,8 | 23,4 |
| 25,4 | 34,3 | 36,9 | 39,2 |
| - | 47,9 | 51,6 | 52,8 |
| 54,8 | 56,3 | 57,2 | 57,9 |
| 60,9 | | 61,6 | 62,2 |
| 67,0 | 68,4 | 68,4 | 69,1 |
| 69,6 | 70,4 | 71,1 | 71,8 |
| 72,2 | 76,2 | - | 80,8 |
| 83,3 | 88,1 | 93,6 | 97,4 |
| 99,6 | 99,6 | - | 102,6 |
| 112,4 | 123,4 | 124,4 | 126,4 |
| - | - | 133,4 | - |
| - | - | - | - |
| - | 192,3 | 192,6 | |

f) mit einer Molekülformel: $C_{54} H_{84} N_3 O_{22} S_4$ I; und

g) mit einem Molekulargewicht 1381, herstellbar durch aerobe Fermentation eines flüssigen Mediums, das assimilierbare Quellen für Kohlenstoff, Stickstoff, Jod und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\beta_1$-I durch eine Reihe von Säulenchromatographien und untersuchen und Analysieren der Verbindung LL-E33288$\beta_1$-I mittels Dünnschichtchromatographie.

**12.** Verbindung LL-E33288$\beta_2$-I mit den folgenden $R_f$-Werten in den angegebenen Lösungsmittelsystemen bei Dünnschichtchromatographie auf Silikagel-Platten:

a) Ethylacetat, gesättigt mit 0,1M wässerigem Kaliumdihydrogenphosphat, $R_f$=0,32;

b) 3% Isopropanol in Ethylacecat, gesättigt mit 0,1M wässerigem Kaliumdihydrogenphosphat, $R_f$=0,41; und

c) Ethylacetat:Methanol (95:5), $R_f$=0,45,

herstellbar durch aerobe Fermentation eines flüssigen Mediums, das assimilierbare Quellen für Kohlenstoff, Stickstoff, Jod und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\beta_2$-I durch eine Reihe von Säulenchromatographien und Untersuchen und Analysieren der Verbindung LL-E33288$\beta_2$ -I mittels Dünnschichtchromatographie.

**13.** Verbindung LL-E33288$\gamma_1$-I,

a) mit den folgenden $R_f$-werten in den angegebenen Lösungsmittelsystemen bei Dünnschichtchromatographie auf Silikagel-Platten:

i) Ethylacetat, gesättigt mit 0,1M wässerigem Kaliumdihydrogenphosphat, $R_f$=0,18;

ii) 3% Isopropanol in Ethylacetat, gesättigt mit 0,1M wässerigem Kaliumdihydrogenphosphat $R_f$=0,28; und

iii) Ethylacetat:Methanol (95:5), $R_f$=0,27;

b) welches nur die folgenden Elemente enthält: C, H, N, O, S und I;

c) mit einer annähernden Elementaranalyse: C 48,8; H 5,4; N 2,8; S 9,0; und I 9,2;

d) mit einem Molekulargewicht: 1367;

e) mit einer Molekülformel: $C_{53} H_{82} N_3 O_{22} S_4 I$;

f) mit einem Ultraviolett-Absorptionsspektrum wie in Fig. XV der Zeichnungen gezeigt;

g) mit einem Infrarot-Absorptionsspektrum, wie in Fig. XVI der Zeichnungen gezeigt;

h) mit einem Protonen-Magnetresonanzspektrum, wie in Fig. XVII der Zeichnungen gezeigt; und

i) mit einem Kohlenstoff-13-Magnetresonanzspektrum, wie in Fig. XVIII der Zeichnungen gezeigt, mit signifikanten Peake wie nachfolgend aufgezählt;

| | | | |
|---|---|---|---|
| 14,5(q) | 17,6(q) | 17,6(q) | 18,9(q) |
| - | - | 22,8(q) | - |
| 25,4(q); | 34,1(t) | 37,0(t) | 39,1(t) |
| 42,3(t/s) | - | 51,5(d) | 52,8(q) |
| 54,8(t) | 56,3(q) | 57,2(q) | - |
| 60,4(d) | 60,9(q) | 61,3(t) | 61,7(q) |
| 67,0(d) | 68,4(d) | 68,5(d) | 69,2(d) |
| 69,7(d) | 70,5(d) | 71,1(d) | 71,8(d) |
| 72,1(s) | 75,7(d) | 75,8(d) | 80,9(d) |
| 82,8(s) | 88,1(s) | 93,5(s) | 97,3(d) |
| 99,6(d) | 99,7(d) | 100,8(s) | 102,6(d) |
| - | 123,4(d) | 124,4(d) | 126,2(d) |
| 130,2(s) | 131,0(s) | 133,4(s) | 139,1(s) |
| 143,0(s) | 145,1 | 150,6(s) | 151,5(s) |
| 154,5 | 192,0(s) | 192,5(s), | |

herstellbar durch aerobe Fermentation eines flüssigen Mediums, das assimilierbare Quellen für Kohlenstoff, Stickstoff, Jod und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\gamma_1$ -I durch eine Reihe von Säulenchromatographien und Untersuchen und Analysieren der Verbindung LL-E33288$\gamma_1$ -I mittels Dünnschichtchromatographie.

**14.** Verbindung LL-E33288$\delta_1$-I mit den folgenden $R_f$-werten in den angegebenen Lösungsmittelsystemen bei Dünnschichtchromatographie auf Silikagel-Platten:

a) Ethylacetat, gesättigt mit 0,1M wässerigem Kaliumdihydrogenphosphat, $R_f$=0,11; und
b) 3% Isopropanol in Ethylacetat, gesättigt mit 0,1M wässerigem Kaliumdihydrogenphosphat, $R_f$=0,19,

herstellbar durch aerobe Fermentation eines flüssigen Mediums, das assimilierbare Quellen für Kohlenstoff, Stickstoff, Jod und anorganische Salze enthält; welches Medium mit einer lebensfähigen Kultur des Organismus <u>Micromonospora echinospora</u> ssp. <u>calichensis</u> NRRL 15839 oder Mutanten davon, einschließlich NRRL 15975, beimpft ist, Halten der Fermentationskultur auf einer Temperatur von etwa 24 bis 32°C für eine Zeitdauer von etwa 90 bis 200 Stunden, Ernten des breiigen Gemisches und Extrahieren der Antibiotika aus der Fermentationsbrühe mit einem organischen Lösungsmittel und Reinigen der extrahierten Mischung durch selektives Ausfällen aus niedrigen Kohlenwasserstoffen, Abtrennen von LL-E33288$\delta_1$-I durch eine Reihe von Säulenchromatographien und Untersuchen und Analysieren der Verbindung LL-E33288$\delta_1$-I. mittels Dünnschichtchromatographie.

**15.** Verwendung einer Verbindung ausgewählt aus der Gruppe bestehend aus LL-E33288$\alpha_1$-Br; LL-E33288$\alpha_1$ -I; LL-E33288$\alpha_2$ -Br; LL-E33288$\alpha_2$-I; LL-E33288$\alpha_3$ -Br; LL-E33288$\alpha_3$ -I; LL-E33288$\alpha_4$-Br; LL-E33288$\beta_1$ -Br; LL-E33288$\beta_1$ -I; LL-E33288$\beta_2$ -Br; LL-E33288$\beta_2$ -I; LL-E33288$\gamma_1$ -Br; LL-E33288$\gamma_1$ -I; und LL-E33288$\delta_1$ -I zur Herstellung von Medikamenten zur Behandlung bakterieller Infektionen bei Warmblütern.

**16.** Verwendung einer Verbindung ausgewählt aus der Gruppe bestehend aus LL-E33288$\alpha_1$-Br; LL-E33288$\alpha_1$-I; LL-E33288$\alpha_2$-Br; LL-E33288$\alpha_2$-I; LL-E33288$\alpha_3$-Br; LL-E33288$\alpha_3$ -I; LL-E33288$\alpha_4$ -Br; LL-E33288$\beta_1$ -Br; LL-E33288$\beta_1$ -I; LL-E33288$\beta_2$ -Br; LL-E33288$\beta_2$ -I; LL-E33288$\gamma_1$ -Br; LL-E33288$\gamma_1$-I; und LL-E33288$\delta_1$ -I zur Herstellung von Medikamenten zur Hemmung des Wachstums von Tumoren bei einem Säuger.

**17.** Verwendung einer verbindung ausgewählt aus der Gruppe bestehend aus LL-E33288$\alpha_1$ -Br; LL-E33288$\alpha_1$ -I; LL-E33288$\alpha_2$ -Br; LL-E33288$\alpha_2$ -I; LL-E33288$\alpha_3$ -Br; LL-E33288$\alpha_3$-I; LL-E33288$\alpha_4$ -Br; LL-E33288$\beta_1$ -Br; LL-E33288$\beta_1$ -I; LL-E33288$\beta_2$ -Br; LL-E33288$\beta_2$ -I; LL-E33288$\gamma_1$ -Br; LL-E33288$\gamma_1$ -I; und LL-E33288$\delta_1$ -I zur Herstellung von Medikamenten zur Regression von Leukämie bei einem Säuger.

**18.** Verfahren zur Herstellung der Antibiotika LL-E33288$\alpha_1$ -Br; LL-E33288$\alpha_2$ -Br; LL-E33288$\alpha_3$ -Br; LL-E33288$\alpha_4$ -Br; LL-E33288$\beta_1$ -Br; LL-E33288$\beta_2$ -Br und LL-E33288$\gamma_1$ -Br, welches die aerobe Fermentation des Organismus <u>Micromonospora echinospora</u> ssp. <u>calichensis</u> NRRL 15839 oder Mutanten davon, einschließlich NRRL 15975, in einem flüssigen Medium enthaltend assimilierbare Quellen für Kohlenstoff, Stickstoff, Brom und anorganische Salze, bis dem Medium eine im wesentlichen antibiotische Aktivität verliehen ist, und die nachfolgende Gewinnung der Antibiotika aus diesem umfaßt.

**19.** Verfahren zur Herstellung der Antibiotika LL-E33288$\alpha_1$ -Br; LL-E33288$\alpha_2$ -Br; LL-E33288$\alpha_3$, -Br; LL-E33288$\alpha_4$ -Br; LL-E33288$\beta_1$ -Br; LL-E33288$\beta_2$ -Br und LL-E33288$\gamma_1$ -Br, welches die aerobe Fermentation eines flüssigen Mediums, das assimilierbare Quellen für Kohlenstoff, Stickstoff, Brom und anorganische Salze enthält, wobei das Medium mit einer lebens fähigen Kultur des Organismus <u>Micromonospora echinospora</u> ssp. <u>calichensis</u> NRRL 15839 oder Mutanten davon, einschließlich NRRL 15975, beimpft ist, das Halten der Fermentationskultur auf einer Temperatur von etwa 24-32°C für eine Zeitdauer von etwa 90-200 Stunden, Ernten des breiigen Gemisches und Extraktion der Antibiotika umfaßt.

**20.** Verfahren zur Herstellung der Antibiotika LL-E33288$\alpha_1$ -I; LL-E33288$\alpha_2$ -I; LL-E33288$\alpha_3$ -I; LL-E33288$\beta_1$ -I; LL-E33288$\beta_2$ -I; LL-E33288$\gamma_1$ -I; und LL-E33288$\delta_1$ -I, welches die aerobe Fermentation des Organismus <u>Micromonospora echinospora</u> ssp. <u>calichensis</u> NRRL 15839 oder von Mutanten desselben, einschließlich NRRL 15975, in einem flüssigen Medium, das assimilierbare Quellen für Kohlenstoff, Stickstoff, Jod und anorganische Salze enthält, bis dem Medium im wesentlichen antibiotische Aktivität verliehen worden ist, und nachfolgende Gewinnung der Antibiotika aus diesem umfaßt.

**21.** Verfahren zur Herstellung der Antibiotika LL-E33288$\alpha_1$ -I; LL-E33288$\alpha_2$ -I; LL-E33288$\alpha_3$-I; LL-E33288$\beta_1$ -I; LL-E33288$\beta_2$ -I; LL-E33288$\gamma_1$ -I; und LL-E33288$\delta_1$ -I, welches die aerobe Fermentation eines flüssigen Mediums, das assimilierbare Quellen für Kohlenstoff, Stickstoff, Jod und anorganische Salze enthält, wobei das Medium mit einer lebensfähigen Kultur des Mikroorganismus <u>Micromonospora echinospora</u> ssp. <u>calichensis</u> NRRL 15839 oder von Mutanten desselben, einschließlich NRRL 15975, beimpft ist, Halten der Fermentationskultur auf einer Temperatur von etwa 24-32°C für eine Zeitdauer von etwa 90-200 Stunden, Ernten des breiigen Gemisches und Extraktion der Antibiotika umfaßt.

**22.** Kultur, die den Mikroorganismus <u>Micromonospora echinospora</u> ssp. <u>calichensis</u> NRRL 15839 aufweist, wobei die Kultur fähig ist, den LL-E33288-Komplex in gewinnbarer Menge nach aerober Fermentation in einem wässerigen Medium, das assimilierbare Quellen für Kohlenstoff, Stickstoff, anorganische Salze und entweder Jod oder Brom oder beides enthält, zu erzeugen.

**23.** Kultur, die den Mikroorganismus <u>Micromonospora echinospora</u> ssp. <u>calichensis</u> NRRL 15975 aufweist, wobei die Kultur fähig ist, den LL-E33288-Komplex in gewinnbarer Menge nach aerober Fermentation in einem wässerigen Medium, das assimilierbare Quellen für Kohlenstoff, Stickstoff, anorganische Salze und entweder Jod oder Brom oder beides enthält, zu erzeugen.

**Revendications**

**1.** Composé LL-E33288$\alpha_1$-Br, ayant les valeurs de $R_f$ suivantes dans les systèmes de solvants indiqués dans des TLC sur feuilles de gel de silice :

a) acétate d'éthyle saturé avec du dihydrogénophosphate de potassium aqueux 0,1 M, $R_f$ = 0,67;
b) isopropanol à 3% dans de l'acétate d'éthyle saturé avec du dihydrogénophosphate de potassium aqueux 0,1 M, $R_f$ = 0,80, et
c) acétate d'éthyle:méthanol (95:5), $R_f$ = 0,79, pouvant être produit par fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\alpha_1$-Br par une série de chromatographies sur colonne et par dosage et analyse du composé LL-E33288$\alpha_1$-Br par chromatographie en couche mince.

**2.** Composé LL-E33288$\alpha_2$-Br, ayant les valeurs de $R_f$ suivantes dans les systèmes de solvants indiqués dans des TLC sur feuilles de gel de silice :

a) acétate d'éthyle saturé avec du dihydrogénophosphate de potassium aqueux 0,1 M, $R_f$ = 0,61;
b) isopropanol à 3% dans de l'acétate d'éthyle saturé avec du dihydrogénophosphate de potassium aqueux 0,1 M, $R_f$ = 0,75, et
c) acétate d'éthyle:méthanol (95:5), $R_f$ = 0,73, pouvant être produit par fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp, <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\alpha_2$-Br par une série de chromatographies sur colonne et par dosage et analyse du composé LL-E33288$\alpha_2$-Br par chromatographie en couche mince.

**3.** Composé LL-E33288$\alpha_3$-Br, ayant les valeurs de $R_f$ suivantes dans les systèmes de solvants indiqués dans des TLC sur feuilles de gel de silice :

a) acétate d'éthyle saturé avec du dihydrogénophosphate de potassium aqueux 0,1 M, $R_f$ = 0,55;
b) isopropanol à 3% dans de l'acétate d'éthyle saturé avec du dihydrogénophosphate de potassium aqueux 0,1 M, $R_f$ = 0,69, et
c) acétate d'éthyle:méthanol (95:5), $R_f$ = 0,61, pouvant être produit par fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp. <u>calichensis</u> NRRL 15839

ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\alpha_3$-Br par une série de chromatographies sur colonne et par dosage et analyse du composé LL-E33288$\alpha_3$-Br par chromatographie en couche mince.

**4.** Composé LT-E33288$\alpha_4$-Br, ayant les valeurs de $R_f$ suivantes dans les systèmes de solvants indiqués dans des TLC sur feuilles de gel de silice ;

a) acétate d'éthyle saturé avec du dihydrogénophosphate de potassium aqueux 0,1 M, $R_f$ = 0,49;
b) isopropanol à 3% dans de l'acétate d'éthyle saturé avec du dihydrogénophosphate de potassium aqueux 0,1 M, $R_f$ = 0,64, et
c) acétate d'éthyle:méthanol (95:5), $R_f$ = 0,54, pouvant être produit par fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\alpha_4$-Br par une série de chromatographies sur colonne et par dosage et analyse du composé LL-E33288$\alpha_4$-Br par chromatographie en couche mince.

**5.** Composé LL-E33288$\beta_1$-Br,

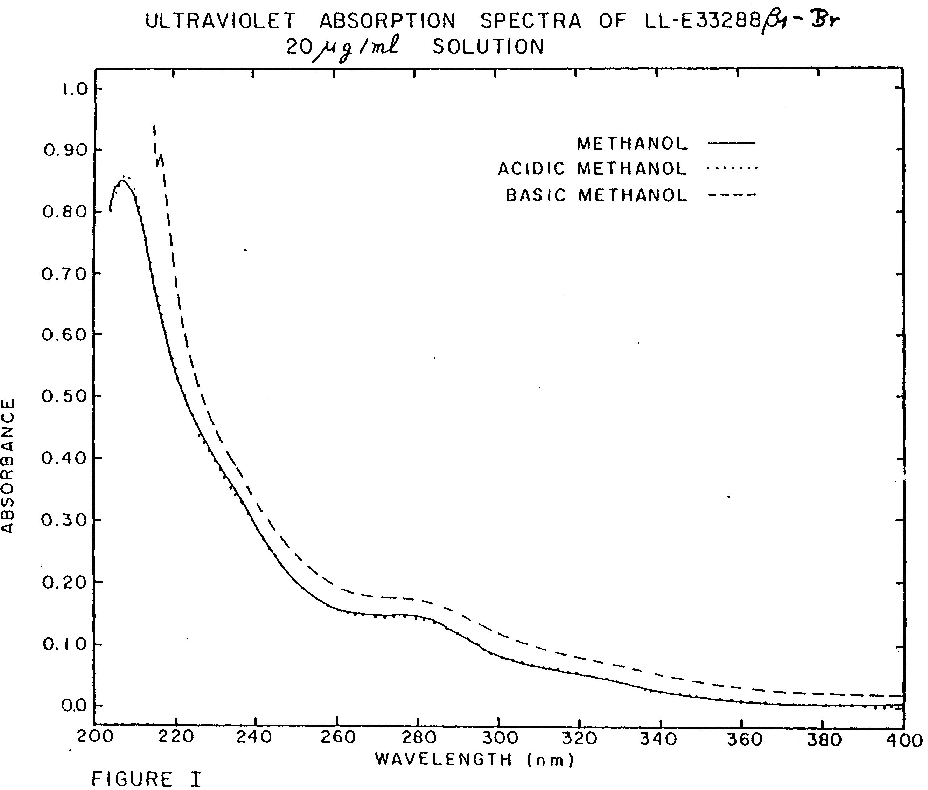

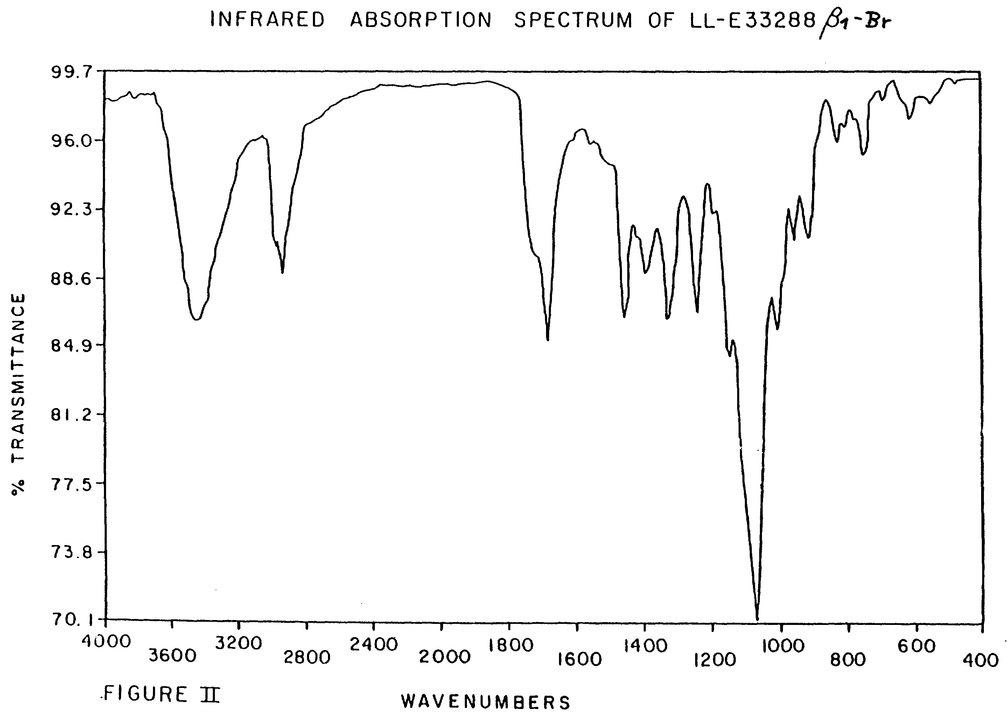

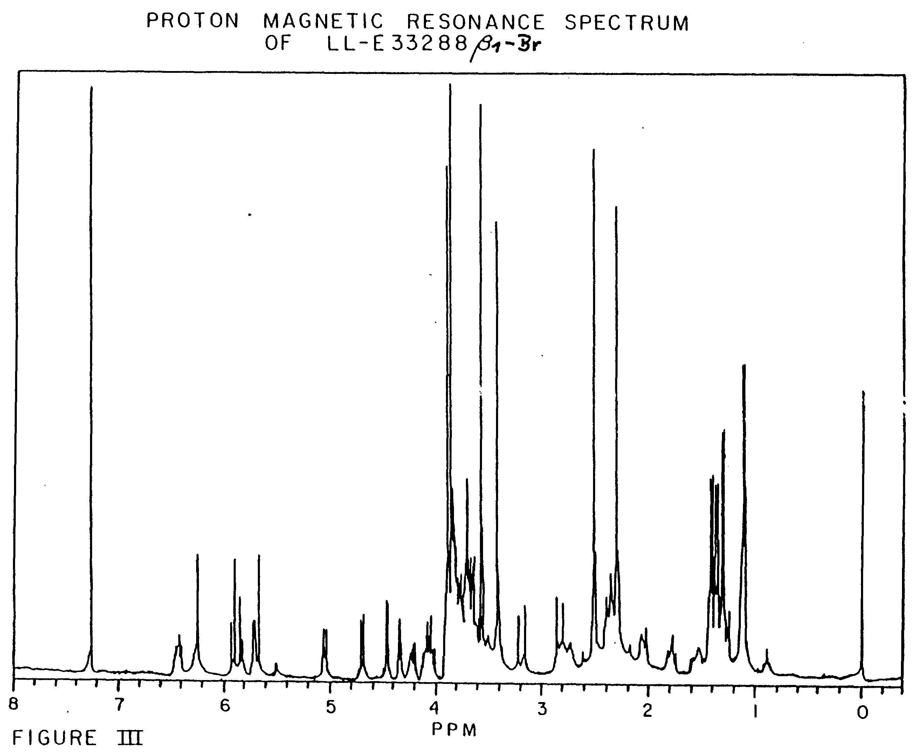

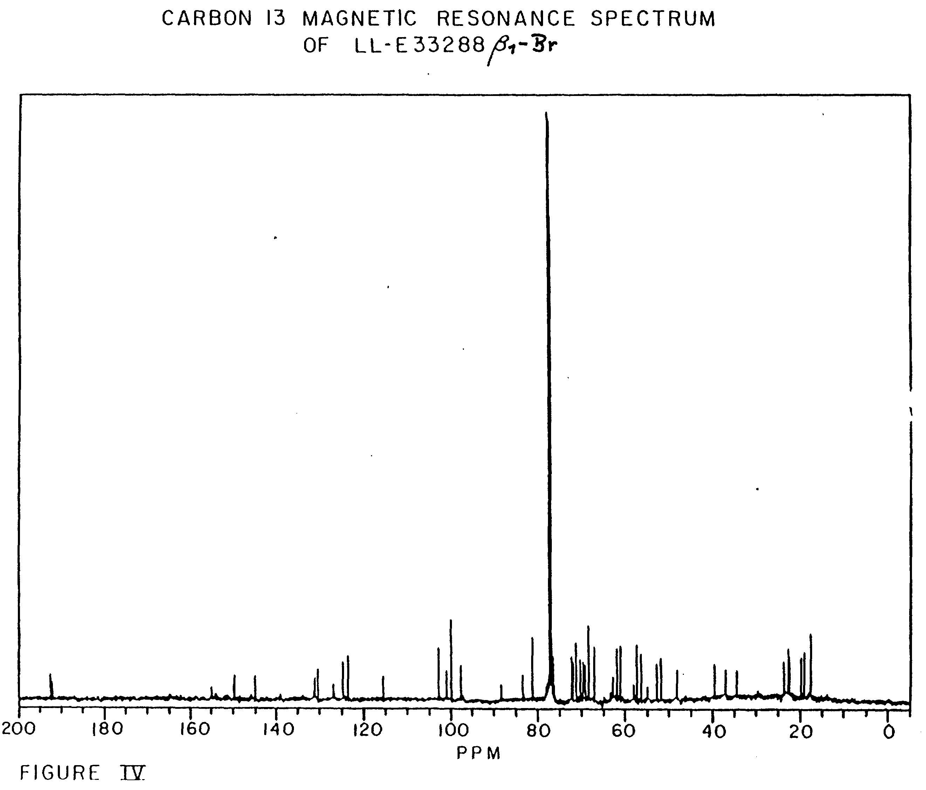

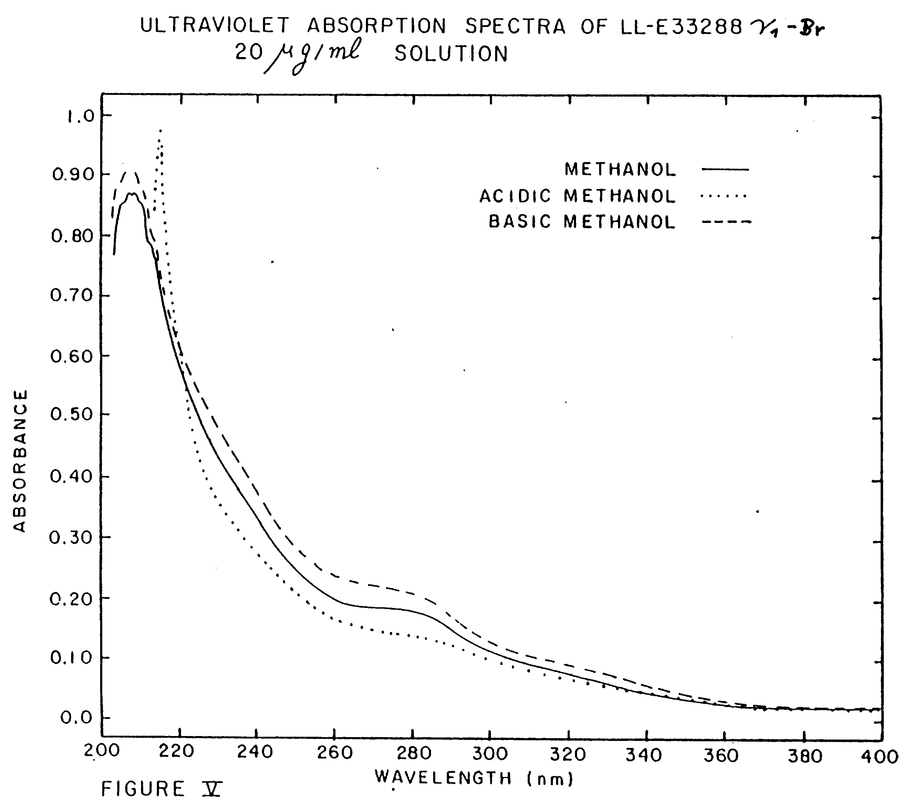

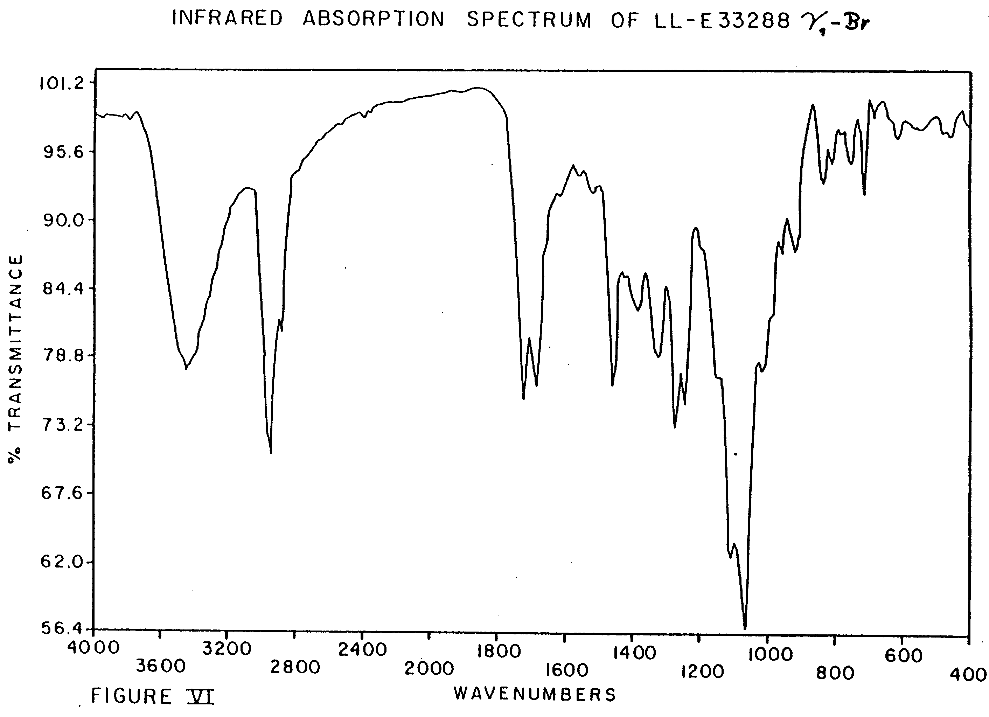

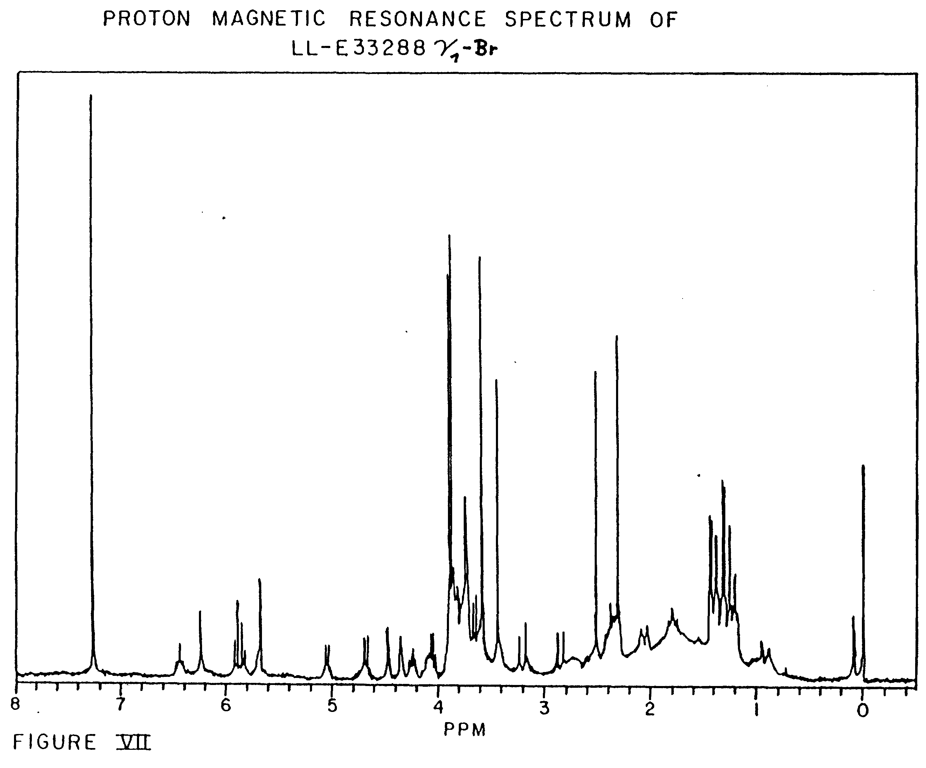

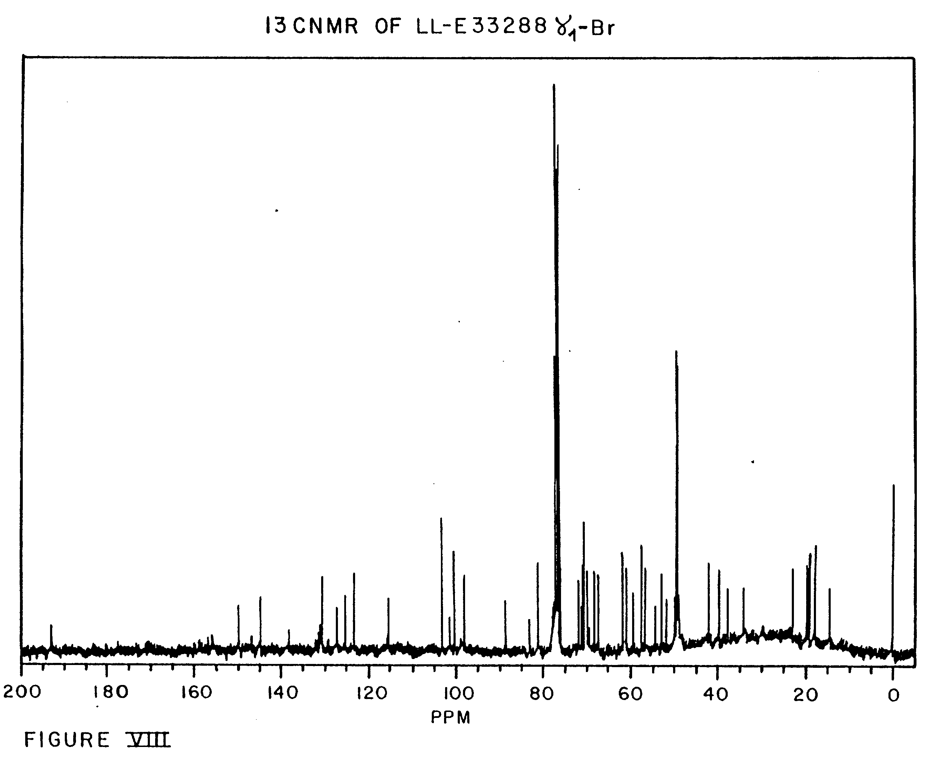

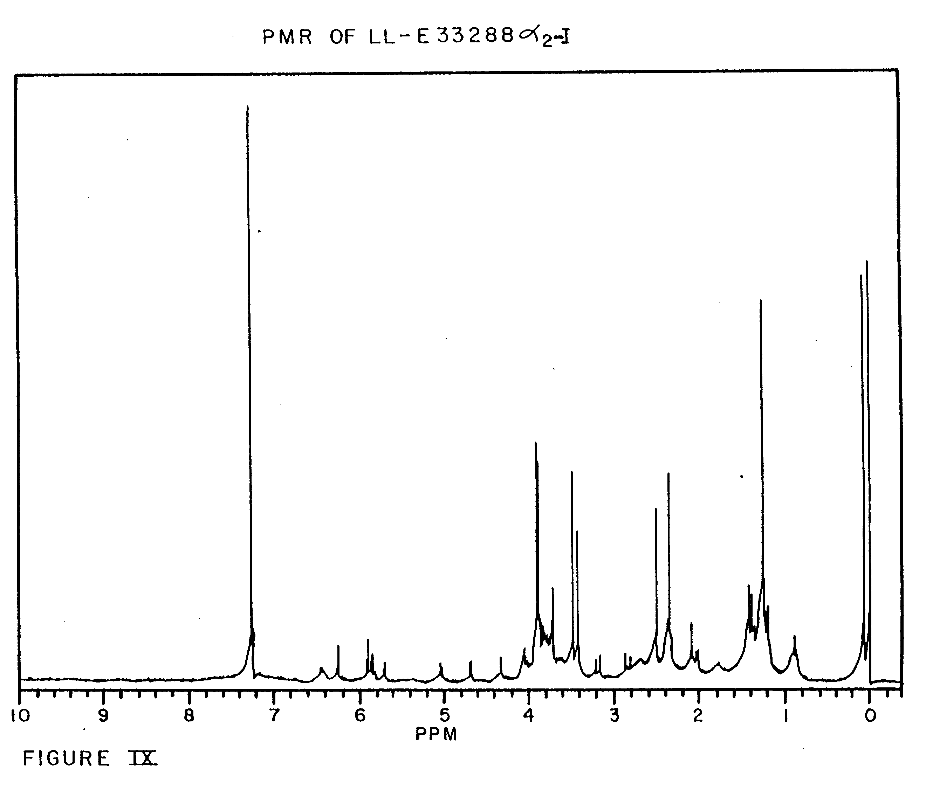

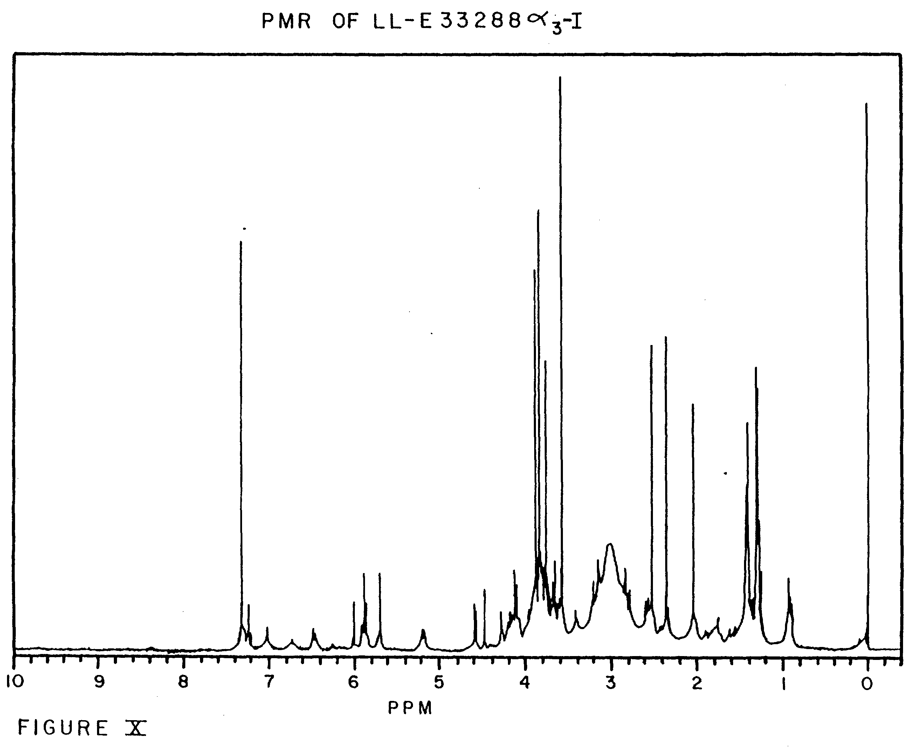

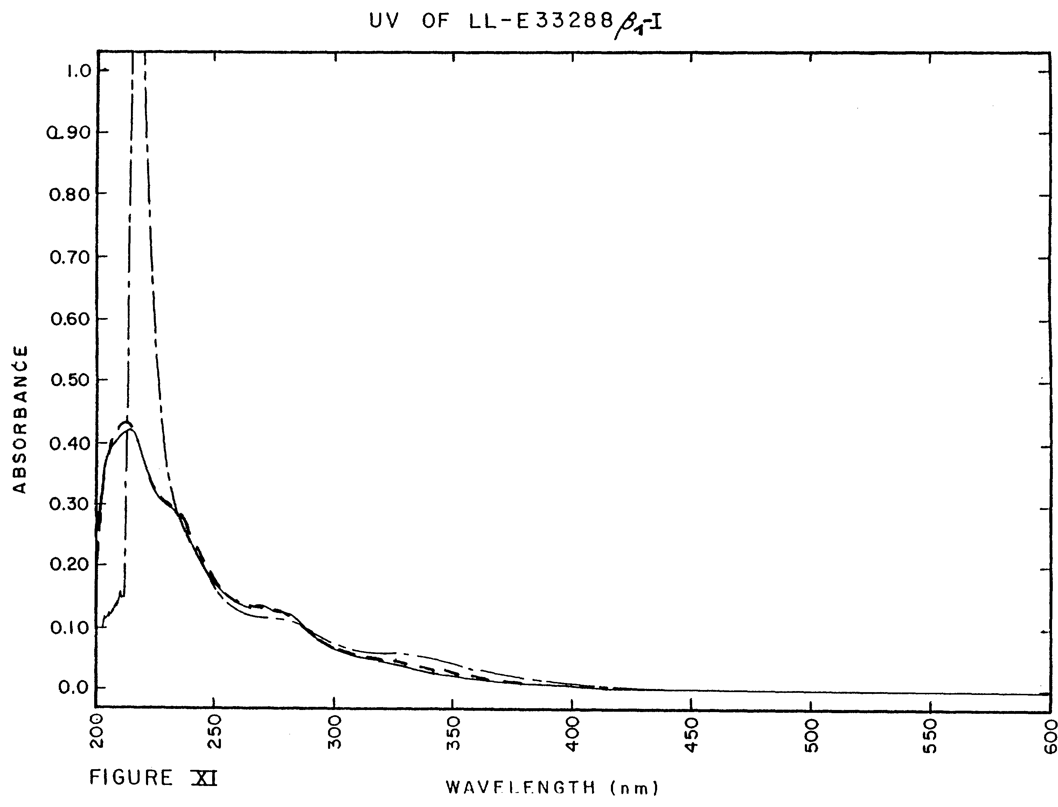

a) ayant une analyse élémentaire approximative :
C 48,6; H 5,6; N 2,9; S 9,1 et Br 5,5;
b) ayant un point de fusion : 146-150°C (déc.);
c) ayant un pouvoir rotatoire spécifique : $[\alpha]_D^{26}$ = -49±10° (0,1%, éthanol);
d) ayant un spectre d'absorption dans l'ultraviolet comme le montre la Fig. 1 des dessins;
e) ayant un spectre d'absorption dans l'infrarouge comme le montre la Fig. 2 des dessins;
f) ayant un spectre de résonance magnétique nucléaire du proton comme le montre la Fig, 3 des dessins;
g) ayant un spectre de résonance magnétique nucléaire du carbone 13 comme le montre la Fig. 4 des dessins, avec des pics significatifs à :

| | | | |
|---|---|---|---|
| 17,60(q); | 17,64(q); | 18,9(q); | 19,7(q); |
| 22,4(q); | 22,8(q); | 23,5(q); | 34,3(t); |
| 36,9(t); | 39,2(t/d); | 47,8(d); | 51,7(q); |
| 52,7(q); | 54,6(d); | 56,3(q); | 57,2(q); |
| 57,8(d); | 61,0(q/d); | 61,7(d); | 62,4(t); |
| 66,9(d); | 68,4(d); | 69,1(d); | 69,7(d); |
| 70,2(d); | 71,1(d); | 71,9(d); | 72,1(s); |
| 76,1(d); | 81,0(d); | 83,3(s); | 88,2(s); |
| 97,4(d); | 99,7(d); | 100,8(s); | 102,5(d); |
| 115,1(s); | 123,4(d); | 124,4(d); | 126,5(d); |
| 130,2(s); | 130,8(s); | 144,6(s); | 149,3(s); |
| 149,5(s); | 191,7(s); | 192,4(s); | et |

h) ayant les valeurs de $R_f$ suivantes dans les systèmes de solvants indiqués dans des TLC sur feuilles de gel de silice :

i) acétate d'éthyle saturé avec du dihydrogénophosphate de potassium aqueux 0,1 M, $R_f$ = 0,24;
iI) isopropanol à 3% dans de l'acétate d'éthyle saturé avec du dihydrogénophosphate de potassium aqueux 0,1 M, $R_f$ = 0,35, et
iii) acétate d'éthyle:méthanol (95:5), $R_f$ = 0,36;

i) ayant un poids moléculaire : 1333/1335, respectivement pour $^{79}$Br/$^{81}$Br, et

j) ayant une formule moléculaire : $C_{54}H_{64}N_3O_{22}S_4Br$,

pouvant être produit par fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp, <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\beta_1$-Br par une série de chromatographies sur colonne et par dosage et analyse du composé LL-E33288$\beta_1$-Br par chromatographie en couche mince.

6. Composé LL-E33288$\beta_2$-Br, ayant les valeurs de $R_f$ suivantes dans les systèmes de solvants indiqués dans des TLC sur feuilles de gel de silice :

a) acétate d'éthyle saturé avec du dihydrogénophosphate de potassium aqueux 0,1 M, $R_f$ = 0,32;

b) isopropanol à 3% dans de l'acétate d'éthyle saturé avec du dihydrogénophosphate de potassium aqueux 0,1 M, $R_f$ = 0,41, et

c) acétate d'éthyle:méthanol (95:5), $R_f$ = 0,45, pouvant être produit par fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\beta_2$-Br par une série de chromatographies sur colonne et par dosage et analyse du composé LL-E33288$\beta_2$-Br par chromatographie en couche mince.

7. Composé LL-E33288$\gamma_1$-Br,

a) ayant un spectre d'absorption dans l'ultraviolet comme le montre la Fig. 5 des dessins;

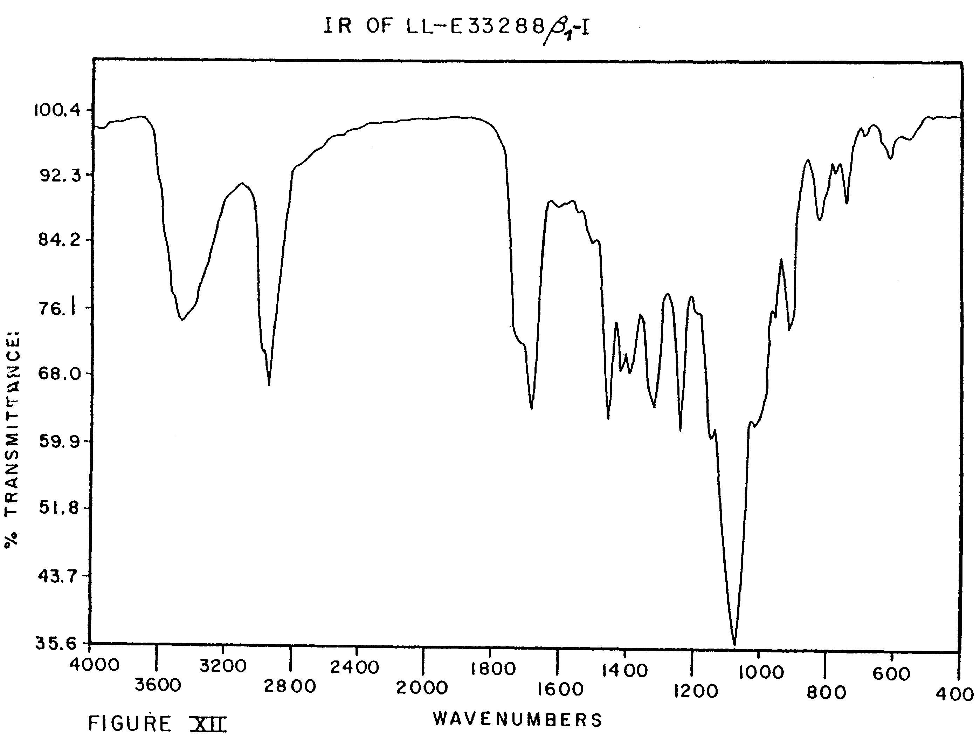

b) ayant un spectre d'absorption dans l'infrarouge comme le montre la Fig. 6 des dessins;

c) ayant un spectre de résonance magnétique nucléaire du proton comme le montre la Fig. 7 des dessins; et

d) ayant les valeurs de $R_f$ suivantes dans les systèmes de solvants indiqués dans des TLC sur feuilles de gel de silice :

i) acétate d'éthyle saturé avec du dihydrogénophosphate de potassium aqueux 0,1 M, $R_f$ = 0,18;

ii) isopropanol à 3% dans de l'acétate d'éthyle saturé avec du dihydrogénophosphate de potassium aqueux 0,1 M, $R_f$ = 0,28, et

iii) acétate d'éthyle:méthanol (95:5), $R_f$ = 0,27;

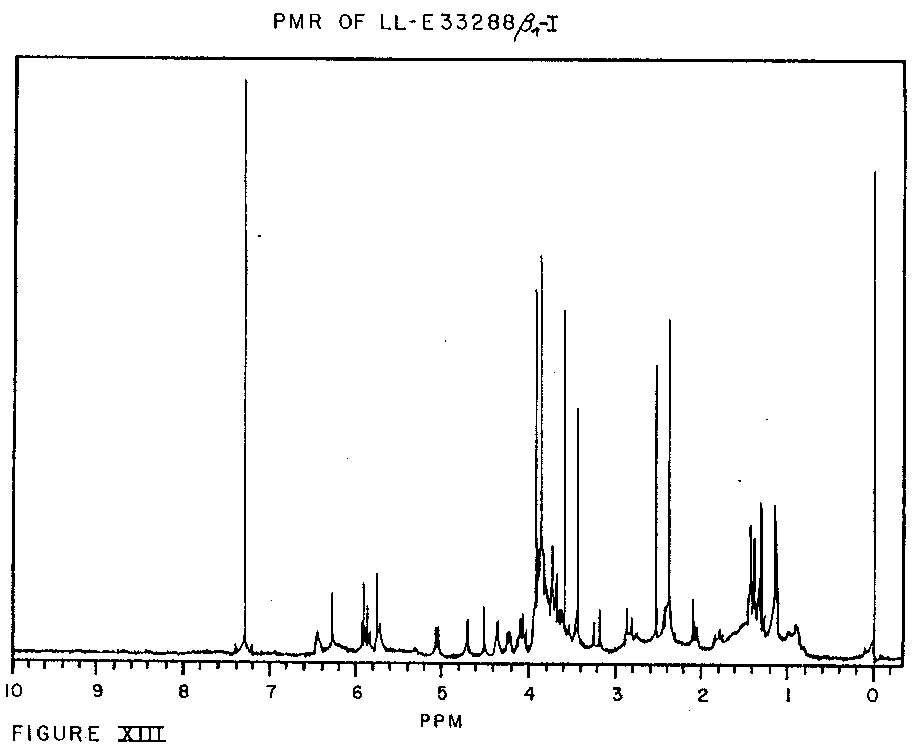

e) ayant un spectre de résonance magnétique nucléaire du carbone 13 comme le montre la Fig. 8 des dessins, avec des pics significatifs à :

| | | | |
|---|---|---|---|
| 14,4 | 17,6 | 17,9 | 19,0 |
| 19,7 | - | 22,8 | - |
| - | 34,0 | 37,6 | 39,5 |
| 42,1 | - | 51,6 | 52,7 |
| 54,1 | 56,3 | 57,3 | - |
| 59,3 | 61,1 | 61,8 | 61,9 |
| 67,2 | 68,18 | 68,23 | 69,7 |
| 70,1 | 70,8 | 71,1 | 71,7 |
| 71,8 | 76,1 | - | 81,0 |
| 82,9 | 88,4 | - | 97,8 |
| 100,0 | 100,2 | 101,3 | 103,0 |
| 115,3 | 123,0 | 124,9 | 126,9 |

(suite)

| | | | |
|---|---|---|---|
| 130,4 | 131,1 | 131,8 | 138,0 |
| 144,7 | - | 149,5 | 149,6 |
| 155,6 | 192,5 | 192,9 | |

f) ayant une formule moléculaire : $C_{55}H_{74}N_3O_{21}S_4Br$, et

g) ayant un poids moléculaire : 1319/1321, respectivement pour $^{79}Br/^{81}Br$,

pouvant être produit par fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\gamma_1$-Br par une série de chromatographies sur colonne et par dosage et analyse du composé LL-E33288$\gamma_1$-Br par chromatographie en couche mince.

**8.** Composé LL-E33288$\alpha$1-I,

a) ayant les valeurs de $R_f$ suivantes dans les systèmes de solvants indiqués dans des TLC sur feuilles de gel de silice :

i) acétate d'éthyle saturé avec du dihydrogénophosphate de potassium aqueux 0,1 M, $R_f = 0,67$;

ii) isopropanol à 3% dans de l'acétate d'éthyle saturé avec du dihydrogénophosphate de potassium aqueux 0,1 M, $R_f = 0,80$, et

iii) acétate d'éthyle:méthanol (95:5), $R_f = 0,80$, et

b) ayant un poids moléculaire de 1145,

pouvant être produit par fermentation aérobie d'un milieu liquide contenant des sources assimilables de carbone, d'azote, d'iode et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\alpha_1$-I par une série de chromatographies sur colonne et par dosage et analyse du composé LL-E33288$\alpha_1$-I par chromatographie en couche mince.

**9.** Composé LL-E33288$\alpha_2$-I,

a) ayant les valeurs de $R_f$ suivantes dans les systèmes de solvants indiqués dans des TLC sur feuilles de gel de silice :

i) acétate d'éthyle saturé avec du dihydrogénophosphate de potassium aqueux 0,1 M, $R_f = 0,61$;

ii) isopropanol à 3% dans de l'acétate d'éthyle saturé avec du dihydrogénophosphate de potassium aqueux 0,1 M, $R_f = 0,75$, et

iii) acétate d'éthyle:méthanol (95:5), $R_f = 0,73$,

b) ne contenant que les éléments suivants : C, H, N, O, S et I;

c) ayant un poids moléculaire de 1131, et

d) ayant un spectre de résonance magnétique nucléaire

du proton comme le montre la Fig. 9 des dessins, pouvant être produit par fermentation aérobie d'un milieu liquide contenant des sources assimilables de carbone, d'azote, d'iode et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\alpha_2$-I par une série de chromatographies sur colonne et par dosage et analyse du composé LL-E33288$\alpha_2$-I par chromatographie en couche mince.

**10.** Composé LL-E33288$\alpha_3$-I,

a) ayant les valeurs de $R_f$ suivantes dans les systèmes de solvants indiqués dans des TLC sur feuilles de gel de silice :

i) acétate d'éthyle saturé avec du dihydrogénophosphate de potassium aqueux 0,1 M, $R_f = 0,55$;
ii) isopropanol à 3% dans de l'acétate d'éthyle saturé avec du dihydrogénophosphate de potassium aqueux 0,1 M, $R_f = 0,69$, et
iii) acétate d'éthyle:méthanol (95:5), $R_f = 0,61$;

b) ayant un poids moléculaire de 1066, et
c) ayant un spectre de résonance magnétique nucléaire

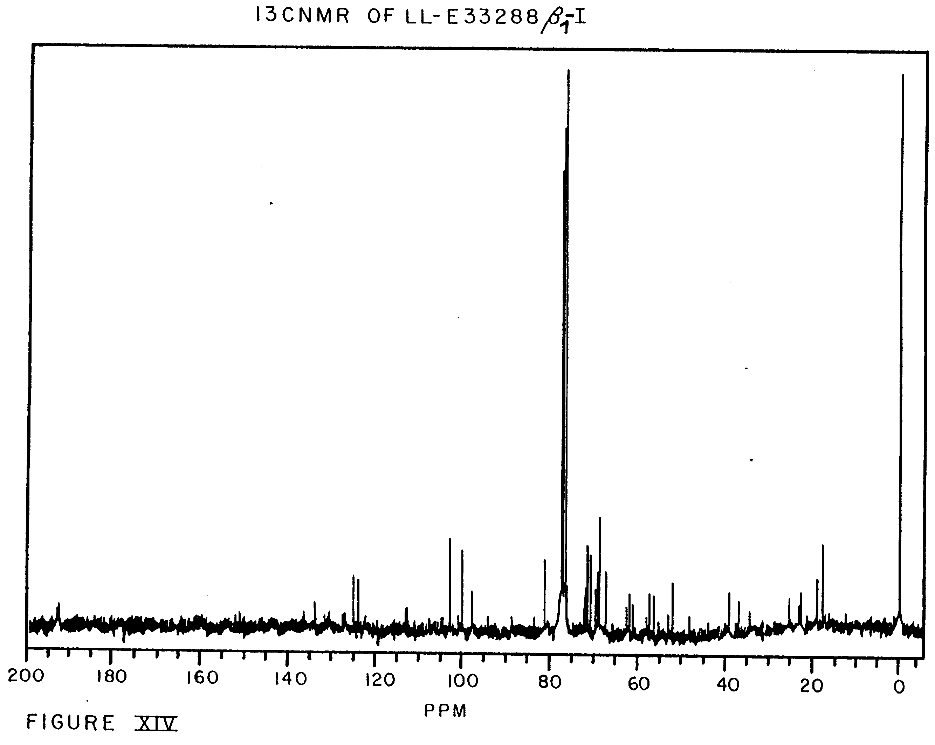

du proton comme le montre la Fig. 10 des dessins, pouvant être produit par fermentation aérobie d'un milieu liquide contenant des sources assimilables de carbone, d'azote, d'iode et de sels inorganiques, ce milieu ayant été inoculé avec une culture viable de l'organisme _Micromonospora echinospora_ spp. _calichensis_ NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\alpha_3$-I par une série de chromatographies sur colonne et par dosage et analyse du composé LL-E33288$\alpha_1$-I par chromatographie en couche mince.

**11.** Composé LL-E33288$\beta_1$-I,

a) ayant les valeurs de $R_f$ suivantes dans les systèmes de solvants indiqués dans des TLC sur feuilles de gel de silice :

i) acétate d'éthyle saturé avec du dihydrogénophosphate de potassium aqueux 0,1 M, $R_f = 0,24$;
ii) isopropanol à 3% dans de l'acétate d'éthyle saturé avec du dihydrogénophosphate de potassium aqueux 0,1 M, $R_f = 0,35$, et
iii) acétate d'éthyle:méthanol (95:5), $R_f = 0,36$;

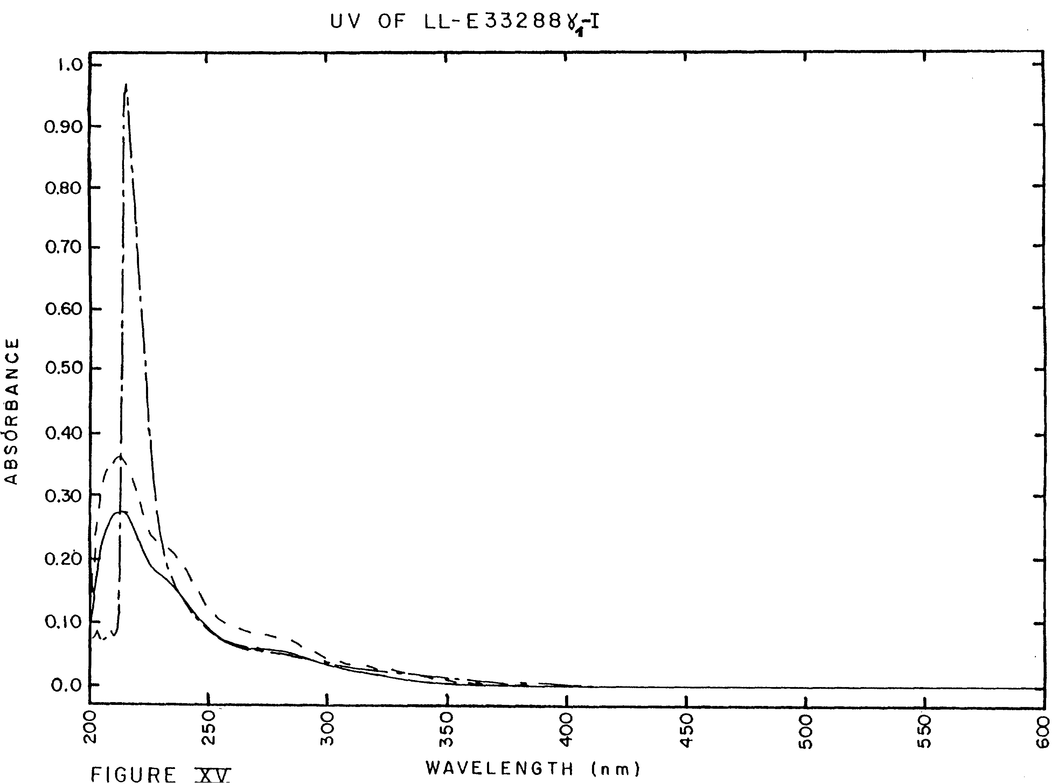

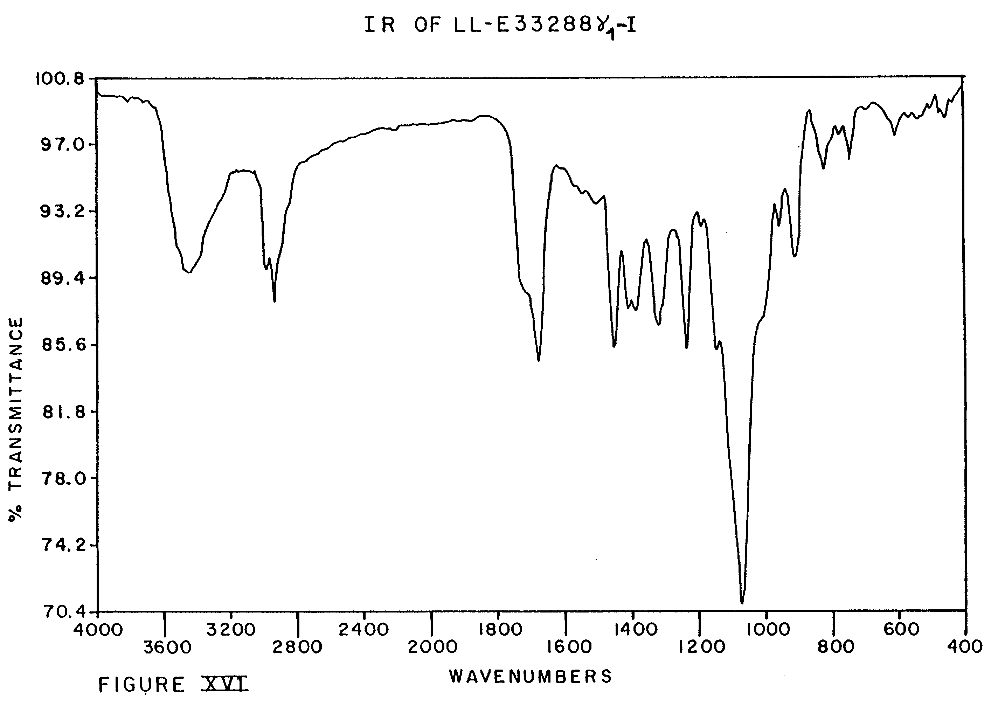

b) ayant un spectre d'absorption dans l'ultraviolet comme le montre la Fig. 11 des dessins;
c) ayant un spectre d'absorption dans l'infrarouge comme le montre la Fig. 12 des dessins;
d) ayant un spectre de résonance magnétique nucléaire du proton comme le montre la Fig. 13 des dessins;
e) ayant un spectre de résonance magnétique nucléaire du carbone 13 comme le montre la Fig. 14 des dessins, avec des pics significatifs à :

| | | | |
|---|---|---|---|
| - | 17,5 | 17,6 | 18,9 |
| - | 22,4 | 22,8 | 23,4 |
| 25,4 | 34,3 | 36,9 | 39,2 |
| - | 47,9 | 51,6 | 52,8 |
| 54,8 | 56,3 | 57,2 | 57,9 |
| 60,9 | | 61,6 | 62,2 |
| 67,0 | 68,4 | 68,4 | 69,1 |
| 69,6 | 70,4 | 71,1 | 71,8 |
| 72,2 | 76,2 | - | 80,8 |
| 83,3 | 88,1 | 93,6 | 97,4 |
| 99,6 | 99,6 | - | 102,6 |

(suite)

| 112,4 | 123,4 | 124,4 | 126,4 |
|-------|-------|-------|-------|
| - | - | 133,4 | - |
| - | - | - | - |
| - | 192,3 | 192,6 | |

f) ayant une formule moléculaire : $C_{54}H_{84}N_3O_{22}S_4I$, et
b) ayant un poids moléculaire de 1381,

pouvant être produit par fermentation aérobie d'un milieu liquide contenant des sources assimilables de carbone, d'azote, d'iode et de sels inorganiques, ce milieu ayant été inoculé avec une culture viable de l'organisme Micromonospora echinospora spp. calichensis NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\beta_1$-I par une série de chromatographies sur colonne et par dosage et analyse du composé LL-E33288$\beta_1$-I par chromatographie en couche mince.

**12.** Composé LL-E33288$\beta_2$-I, ayant les valeurs de $R_f$ suivantes dans les systèmes de solvants indiqués dans des TLC sur feuilles de gel de silice :

a) acétate d'éthyle saturé avec du dihydrogénophosphate de potassium aqueux 0,1 M, $R_f$ = 0,32;
b) isopropanol à 3% dans de l'acétate d'éthyle saturé avec du dihydrogénophosphate de potassium aqueux 0,1 M, $R_f$ = 0,41, et
c) acétate d'éthyle:méthanol (95:5), $R_f$ = 0,45,

pouvant être produit par fermentation aérobie d'un milieu liquide contenant des sources assimilables de carbone, d'azote, d'iode et de sels inorganiques, ce milieu ayant été inoculé avec une culture viable de l'organisme Micromonospora echinospora spp. calichensis NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\beta_2$-I par une série de chromatographies sur colonne et par dosage et analyse du composé LL-E33288$\beta_2$-I par chromatographie en couche mince.

**13.** Composé LL-E33288$\gamma_1$-I,

a) ayant les valeurs de $R_f$ suivantes dans les systèmes de solvants indiqués dans des TLC sur feuilles de gel de silice :

i) acétate d'éthyle saturé avec du dihydrogénophosphate de potassium aqueux 0,1 M, $R_f$ = 0,18;
ii) isopropanol à 3% dans de l'acétate d'éthyle saturé avec du dihydrogénophosphate de potassium aqueux 0,1 M, $R_f$ = 0,28, et
iii) acétate d'éthyle:méthanol (95:5), $R_f$ = 0,27,

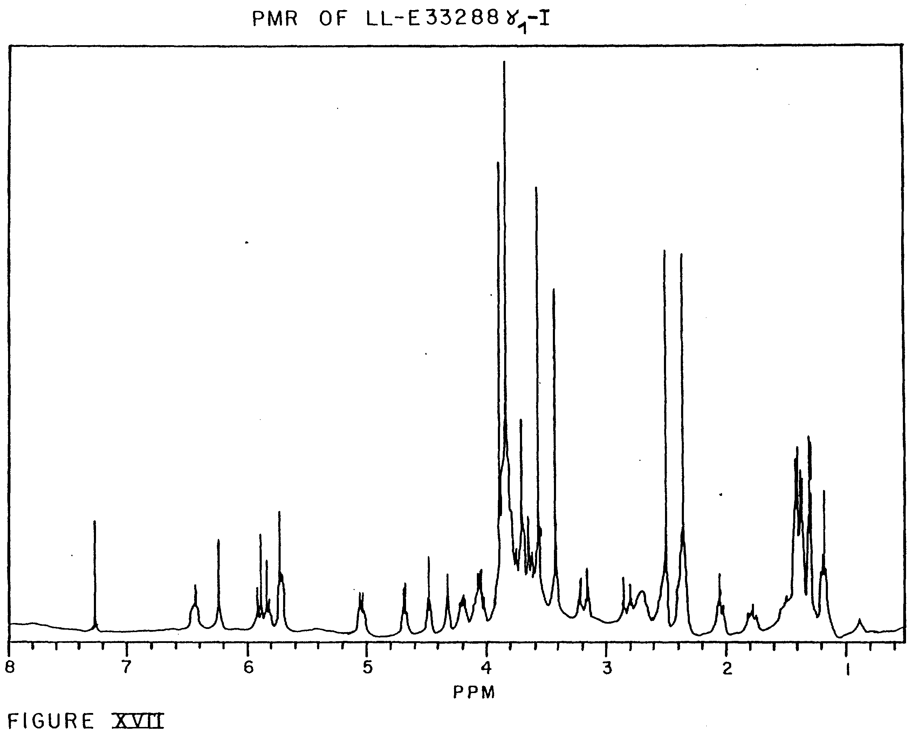

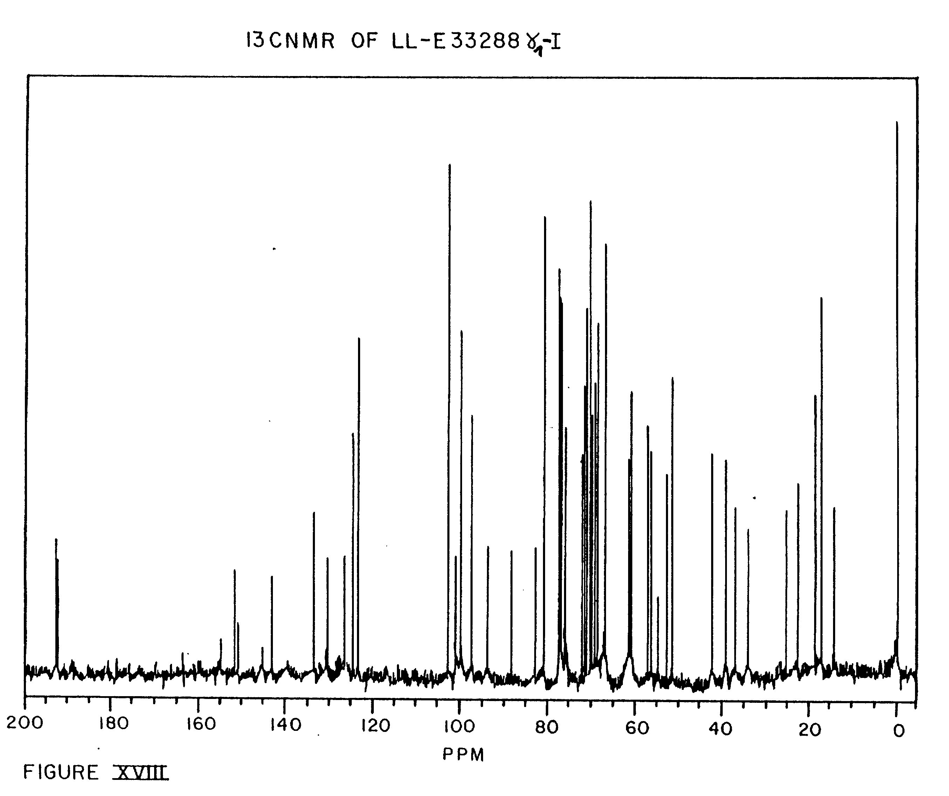

b) ne contenant que les éléments suivants : C, H, N, O, S et I;
c) ayant une analyse élémentaire approximative : C 48,8; H 5,4; N 2,8; S 9,0 et I 9,2;
d) ayant un poids moléculaire de 1367;
e) ayant une formule moléculaire : $C_{53}H_{82}N_3O_{22}S_4I$;
f) ayant un spectre d'absorption dans l'ultraviolet comme le montre la Fig. 15 des dessins;
g) ayant un spectre d'absorption dans l'infrarouge comme le montre la Fig. 16 des dessins;
h) ayant un spectre de résonance magnétique nucléaire du proton comme le montre la Fig. 17 des dessins; et
i) ayant un spectre de résonance magnétique nucléaire du carbone 13 comme le montre la Fig. 18 des dessins, avec des pics significatifs à :

| 14,5(q) | 17,6(q) | 17,6(q) | 18,9(q) |
|---------|---------|---------|---------|
| - | - | 22,8(q) | - |
| 25,4(q) | 34,1(t) | 37,0(t) | 39,1(t) |
| 42,3(t/s) | - | 51,5(d) | 52,8(q) |
| 54,8(t) | 56,3(q) | 57,2(q) | - |
| 60,4(d) | 60,9(q) | 61,3(t) | 61,7(q) |
| 67,0(d) | 68,4(d) | 68,5(d) | 69,2(d) |
| 69,7(d) | 70,5(d) | 71,1(d) | 71,8(d) |
| 72,1(s) | 75,7(d) | 75,8(d) | 80,9(d) |
| 82,8(s) | 88,1(s) | 93,5(s) | 97,3(d) |
| 99,6(d) | 99,7(d) | 100,8(s) | 102,6(d) |
| - | 123,4(d) | 124,4(d) | 126,2(d) |
| 130,2(s) | 131,0(s) | 133,4(s) | 139,1(s) |
| 143,0(s) | 145,1 | 150,6(s) | 151,5(s) |
| 154,5 | 192,0(s) | 192,5(s) | |

pouvant être produit par fermentation aérobie d'un milieu liquide contenant des sources assimilables de carbone, d'azote, d'iode et de sels inorganiques, ce milieu ayant été inoculé avec une culture viable de l'organisme <u>Micromonospora echinospora</u> spp, <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\gamma_1$-I par une série de chromatographies sur colonne et par dosage et analyse du composé LL-E33288$\gamma_1$-I par chromatographie en couche mince.

**14.** Composé LL-E33288$\delta_1$-I ayant les valeurs de $R_f$ suivantes dans les systèmes de solvants indiqués dans des TLC sur feuilles de gel de silice :

a) acétate d'éthyle saturé avec du dihydrogénophosphate de potassium aqueux 0,1 M, $R_f = 0,11$, et
b) isopropanol à 3% dans de l'acétate d'éthyle saturé avec du dihydrogénophosphate de potassium aqueux 0,1 M, $R_f = 0,19$,

pouvant être produit par fermentation aérobie d'un milieu liquide contenant des sources assimilables de carbone, d'azote, d'iode et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques du bouillon de fermentation avec un solvant organique et purification du mélange extrait par précipitation sélective dans des hydrocarbures inférieurs, par séparation du LL-E33288$\delta_1$-I par une série de chromatographies sur colonne et par dosage et analyse du composé LL-E33288$\delta_1$-I par chromatographie en couche mince.

**15.** Utilisation d'un composé sélectionné dans le groupe composé de LL-E33288$\alpha_1$-Br; de LL-E33288$\alpha_1$-I; de LL-E33288$\alpha_2$-Br; de LL-E33288$\alpha_2$-I; de LL-E33288$\alpha_3$-Br; de LL-E33288$\alpha_3$-I; de LL-E33288$\alpha_4$-Br; de LL-E33288$\beta_1$-Br; de LL-E33288$\beta_1$-I; de LL-E33288$\beta_2$-Br; de LL-E33288$\beta_1$-I; de LL-E33288$\gamma_1$-Br; de LL-E33288$\gamma_1$-I, et de LL-E33288$\delta_1$-I, pour la fabrication de médicaments destinés au traitement d'infections bactériennes chez les animaux à sang chaud.

**16.** Utilisation d'un composé sélectionné dans le groupe composé de LL-E33288$\alpha_1$-Br; de LL-E33288$\alpha_1$-I; de LL-E33288$\alpha_2$-Br; de LL-E33288$\alpha_2$-I; de LL-E33288$\alpha_3$-Br; de LL-E33288$\alpha_3$-I; de LL-E33288$\alpha_4$-Br; de LL-E33288$\beta_1$-Br; de LL-E33288$\beta_1$-I; de LL-E33288$\beta_2$-Br; de LL-E33288$\beta_2$-I; de LL-E33288$\gamma_1$-Br; de LL-E33288$\gamma_1$-I, et de LL-E33288$\delta_1$-I, pour la fabrication de médicaments destinés à inhiber la croissance de tumeurs chez un mammifère.

**17.** Utilisation d'un composé sélectionné dans le groupe composé de LL-E33288$\alpha_1$-Br; de LL-E33288$\alpha_1$-I; de LL-E33288$\alpha_2$-Br; de LL-E33288$\alpha_2$-I; de LL-E33288$\alpha_3$-Br; de LL-E33288$\alpha_3$-I; de LL-E33288$\alpha_4$-Br; de LL-E33288$\beta_1$-

EP 0 182 152 B1

Br; de LL-E33288$\beta_1$-I; de LL-E33288$\beta_2$-Br; de LL-E33288$\beta_2$-I; de LL-E33288$\gamma_1$-Br; de LL-E33288$\gamma_1$-I, et de LL-E33288$\delta_1$-I, pour la fabrication de médicaments destinés à faire régresser la leucémie chez un mammifère.

18. Procédé de production d'antibiotiques LL-E33288$\alpha_1$-Br, LL-E33288$\alpha_2$-Br, LL-E33288$\alpha_3$-Br, LL-E33288$\alpha_4$-Br, LL-E33288$\beta_1$-Br, LL-E33288$\beta_2$-Br et LL-E33288$\gamma_1$-Br, lequel comprend la fermentation aérobie de l'organisme <u>Micromonospora echinospora</u> spp. <u>calichensis</u> NRRL 15839 ou des mutants de celui-ci, y compris NRRL 15975 dans un milieu liquide contenant des sources assimilables de carbone, d'azote, de brome et de sels inorganiques, jusqu'à ce qu'une activité antibiotique substantielle soit conférée audit milieu, puis la récupération des antibiotiques à partir de celui-ci.

19. Procédé de production d'antibiotiques LL-E33288$\alpha_1$-Br, LL-E33288$\alpha_2$-Br, LL-E33288$\alpha_3$-Br, LL-E33288$\alpha_4$-Br, LL-E33288$\beta_1$-Br, LL-E33288$\beta_2$-Br et LL-E33288$\gamma_1$-Br, lequel comprend la fermentation aérobie d'un milieu liquide contenant des sources assimilables de carbone, d'azote, de brome et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques,

20. Procédé de production d'antibiotiques LL-E332880$\alpha_1$-I, LL-E33288$\alpha_2$-I, LL-E33288$\alpha_3$-I, LL-E33288$\beta_1$-I, LL-E33288$\beta_2$-I, LL-E33288$\gamma_1$-I et LL-E33288$\delta_1$-I, lequel comprend la fermentation aérobie de l'organisme <u>Micromonospora echinospora</u> spp. <u>calichensis</u> NRRL 15839 ou des mutants de celui-ci, y compris NRRL 15975, dans un milieu liquide contenant des sources assimilables de carbone, d'azote, d'iode et de sels inorganiques, jusqu'à ce qu'une activité antibiotique substantielle soit conférée audit milieu, puis la récupération des antibiotiques à partir de celui-ci.

21. Procédé de production d'antibiotiques LL-E33288$\alpha_1$-I, LL-E33288$\alpha_2$-I, LL-E33288$\alpha_3$-I, LL-E33288$\beta_1$-I, LL-E33288$\beta_2$-I, LL-E33288$\gamma_1$-I et LL-E33288$\delta_1$-I, lequel comprend la fermentation aérobie d'un milieu liquide contenant des sources assimilables de carbone, d'azote, d'iode et de sels inorganiques, ce milieu ayant été inoculé avec une culture viable de l'organisme <u>Micromonospora echinospora</u> spp. <u>calichensis</u> NRRL 15839 ou des mutants de celui-ci, y compris NRRL 15975, par maintien de ladite culture en fermentation à une température d'environ 24 à 32°C durant une période d'environ 90 à 200 heures, par récolte de la pâte, et par extraction des antibiotiques.

22. Culture contenant le micro-organisme <u>Micromonospora echinospora</u> spp. <u>calichensis</u>, NRRL 15839, ladite culture étant capable de produire le complexe LL-E33288 en quantité récupérable par fermentation aérobie dans un milieu liquide contenant des sources assimilables de carbone, d'azote, de sels inorganiques, et soit d'iode, soit de brome, soit des deux.

23. Culture contenant le micro-organisme <u>Micromonospora echinospora</u> spp. <u>calichensis,</u> NRRL 15975, ladite culture étant capable de produire le complexe LL-E33288 en quantité récupérable par fermentation aérobie dans un milieu liquide contenant des sources assimilables de carbone, d'azote, de sels inorganiques, et soit d'iode, soit de brome, soit des deux.

47

ULTRAVIOLET ABSORPTION SPECTRA OF LL-E33288$\beta_1$-Br
20 $\mu g$/m$\ell$ SOLUTION

FIGURE I

EP 0 182 152 B1

INFRARED ABSORPTION SPECTRUM OF LL-E33288 $\beta_1$-Br

FIGURE II

WAVENUMBERS

% TRANSMITTANCE

EP 0 182 152 B1

PROTON MAGNETIC RESONANCE SPECTRUM
OF LL-E 33288 β₁-Br

FIGURE III

PPM

EP 0 182 152 B1

CARBON 13 MAGNETIC RESONANCE SPECTRUM
OF LL-E33288 $\beta_1$-Br

PPM

FIGURE IV

EP 0 182 152 B1

ULTRAVIOLET ABSORPTION SPECTRA OF LL-E33288 $\gamma_1$-Br
20 $\mu g/ml$ SOLUTION

METHANOL ———
ACIDIC METHANOL ·······
BASIC METHANOL ─ ─ ─ ─

FIGURE V

EP 0 182 152 B1

INFRARED ABSORPTION SPECTRUM OF LL-E33288 $\gamma_1$-Br

FIGURE VI

EP 0 182 152 B1

EP 0 182 152 B1

PROTON MAGNETIC RESONANCE SPECTRUM OF
LL-E33288 $\gamma_1$-Br

PPM

FIGURE VII

13 CNMR OF LL-E33288 $\gamma'_1$-Br

PPM

FIGURE VIII

EP 0 182 152 B1

PMR OF LL-E33288 $\alpha_3$-I

FIGURE X

EP 0 182 152 B1

UV OF LL-E33288 $\beta_1^{Br}$-I

FIGURE XI

WAVELENGTH (nm)

ABSORBANCE

# IR OF LL-E33288$\beta_1$-I

FIGURE XII

WAVENUMBERS

% TRANSMITTANCE

EP 0 182 152 B1

PMR OF LL-E33288$\beta_1$-I

FIGURE XIII

PPM

13CNMR OF LL-E33288 $\beta_1$-I

PPM

FIGURE XIV

EP 0 182 152 B1

UV OF LL-E33288$\gamma_1$-I

ABSORBANCE

WAVELENGTH (nm)

FIGURE XV

EP 0 182 152 B1

IR OF LL-E33288$\gamma_1$-I

FIGURE XVI

WAVENUMBERS

% TRANSMITTANCE

EP 0 182 152 B1

PMR OF LL-E 33288 $\gamma_1$-I

FIGURE XVII

EP 0 182 152 B1

I3CNMR OF LL-E33288 $\gamma_1$-I

FIGURE XVIII

EP 0 182 152 B1